(19) **Europäisches Patentamt / European Patent Office / Office européen des brevets**

(11) **EP 1 936 370 A1**

(12) **EUROPEAN PATENT APPLICATION**

(43) Date of publication:
**25.06.2008 Bulletin 2008/26**

(51) Int Cl.:
*G01N 30/72* (2006.01)   *G01N 33/50* (2006.01)

(21) Application number: **06026785.3**

(22) Date of filing: **22.12.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Max-Planck-Gesellschaft zur Förderung der Wissenschaften e.V.
80539 München (DE)**

(72) Inventors:
• **Altmann, Thomas
14469 Potsdam (DE)**

• **Willmitzer, Lothar
14129 Berlin (DE)**
• **Selbig, Joachim
14482 Potsdam (DE)**
• **Meyer, Rhonda C.
14476 Potsdam (DE)**
• **Steinfath, Matthias
14059 Berlin (DE)**
• **Lisec, Jan
13053 Berlin (DE)**
• **Fiehn, Oliver
Davis, CA 95616 (US)**

(74) Representative: **Vossius & Partner
Siebertstrasse 4
81675 München (DE)**

(54) **Determination and prediction of the expression of traits of plants from the metabolite profile as a biomarker**

(57)    The present invention relates to a method for determining the correlation between the metabolite profiles (MPs) and the expression or potential for expression of a trait of a group of plants, and further to a method for determining or predicting the expression of a trait of a plant by taking advantage of the determined MP of said plant and a determined correlation between the MPs and the expression or potential for the expression of said trait in a group of plants.

Particularly, the present invention relates to a method for determining or predicting the biomass production/ growth rate of a plant by taking advantage of the determined MP of said plant and a determined correlation between the MPs and the biomass production/growth rate or the potential for biomass production/growth rate of a group of plants.

The present invention also relates to a method for breeding of a plant that takes advantage of a method of determining the expression or potential for expression of a trait of said plant according to any one of the methods as disclosed, and selecting said plant on the basis of the expression or potential for expression of said trait. Furthermore, the present invention relates to a method for identifying quantitative trait loci (QTLs) for a trait or for the potential for expression of a trait of a group of plants comprising the step of identifying QTLs for metabolite combinations showing correlation with the expression or potential for expression of said trait of said group of plants.

The present invention also relates to and a method for identifying a candidate gene involved in the determination of expression or potential for expression of a trait of a plant comprising the step of isolating a gene corresponding to any one of the QTLs as identified by the corresponding method of the present invention.

Additionally, the present invention relates to a method of screening for a plant that exhibits a desired expression or potential for expression of a trait as well as to a method of determining whether a treatment influences the expression or potential for expression of a trait of a plant.

# EP 1 936 370 A1

**Description**

The present invention relates to a method for determining the correlation between the metabolite profiles (MPs) and the expression or potential for expression of a trait of a group of plants, and further to a method for determining or predicting the expression of a trait of a plant by taking advantage of the determined MP of said plant and a determined correlation between the MPs and the expression or potential for the expression of said trait in a group of plants. Particularly, the present invention relates to a method for determining or predicting the biomass production/growth rate of a plant by taking advantage of the determined MP of said plant and a determined correlation between the MPs and the biomass production/growth rate or the potential for biomass production/growth rate of a group of plants.

The present invention also relates to a method for breeding of a plant that takes advantage of a method of determining the expression or potential for expression of a trait of said plant according to any one of the methods as disclosed, and selecting said plant on the basis of the expression or potential for expression of said trait. Furthermore, the present invention relates to a method for identifying quantitative trait loci (QTLs) for a trait or for the potential for expression of a trait of a group of plants comprising the step of identifying QTLs for metabolite combinations showing strong correlation with the expression or potential for expression of said trait of said group of plants.

The present invention also relates to a method for identifying a candidate gene involved in the determination of expression or potential for expression of a trait of a plant comprising the step of isolating a gene corresponding to any one of the QTLs as identified by the corresponding method of the present invention.

Additionally, the present invention relates to a method of screening for a plant that exhibits a desired expression or potential for expression of a trait as well as to a method of determining whether a treatment influences the expression or potential for expression of a trait of a plant.

Multicellular organisms have to adjust their multiplicity of traits (for example their biomass production/growth rate) to a multitude of exogenous and endogenous cues. In order to thrive, they have to optimize the use of available resources to fit their needs in terms of energy, biosynthetic building blocks, and reserves. Unlike animals that satisfy their demand of organic nutrients by feeding on plants or other animals, green plants produce their own organic compounds. Their ability to express certain traits (like, for example, biomass production/growth) thus solely depends on their own photosynthetic and metabolic capacity. The expression of certain traits of a plant, for example the biomass accumulation in the vegetative growth phase, can therefore be regarded as the ultimate expression of the plant's metabolic performance. Plants function as integrated systems in which metabolic and developmental pathways draw on common resource pools and respond to changes in environmental energy and resource supplies (Tonsor, Plant Cell Environ 28, 2-20 (2005)). The metabolic system of a plant therefore has to be well controlled and the distribution of metabolites between several traits, like growth, production of defence compounds, storage compounds, etc., has to be very tightly regulated. For example, growth and the concomitant drain of metabolites into cellular components has to be adjusted to the metabolic capacity of the system i.e. the ability to supply sufficient amounts of organic compounds. This was demonstrated by several observations of growth depression upon reduction of primary metabolism such as sucrose synthesis (Chen, Planta 221, 479-492 (2005)), (Fernie, Planta 214, 510-520 (2002)). Growth ceases upon severe starvation caused by an extended dark period, and is re-initiated only after a lag period of several hours after relief from the starvation by re-illumination (Gibon, Plant Cell 16, 3304-3325 (2004)). Recent observations of the roles of the DELLA proteins in plants indicate that plant growth is limited to a sub-maximum level to enable plants to cope with unfavourable conditions (Achard, Science 311, 91-94 (2006)). Thus, growth rate, but also other traits of a plant, has to be adjusted to the metabolic status of a plant that needs to be translated into an appropriate response. This interaction between metabolism and, for example, the regulatory mechanisms for growth (or other traits) may operate in two ways: Either strong metabolic activity with a high supply of metabolites triggers growth (or other traits), or growth (or other traits) drain (s) metabolites to a minimum tolerable level upon which growth is (or other traits are) restricted. Until now, this question has not been answered with complete satisfaction. Metabolites may exert control on, for example, growth by acting as substrates for the synthesis of cellular components that become limiting under conditions of maximum tolerable growth. Under this scenario growth, or other traits, will be restricted directly by the availability of critical compounds. On the other hand, metabolite levels may be sensed and metabolites may thus play roles as signals of which only few have hitherto been identified. For example, sugars, such as glucose and sucrose have been shown to act as metabolic signals and to be involved in the control of plant growth and development (Gibson, Curr Opin Plant Biol 8, 93-102 (2005)), (Rolland, Biochem Soc T33, 269-271 (2005)). Trehalose-6-phosphate has recently been shown to be involved in signaling of the plant sugar status and in control of growth and development (Avonce, Plant Physiol 136, 3649-3659 (2004)), (Kolbe, P Natl Acad Sci USA 102, 11118-111123 (2005)), (Schluepmann, P Natl Acad Sci USA 100, 6849-6854 (2003)), (Thimm, Plant J 37, 914-939 (2004)).

Comparable to the latter observation, recent research activities mostly focus on simple biomarkers, like individual metabolites, and their role in the physiological network that determines a particular trait.

For many traits of plants, the expression of such simple biomarkers like individual metabolites cannot satisfactorily explain differences in the expression of a trait.. In order to satisfactorily describe certain traits of plants, recently approaches have been suggested that take advantage of comprehensive observations based on plant's transcriptome or proteome (for example, Stokes, 2006, International Sympensium "Heterosis in plants", Potsdam, 18.-20. May 2006). Although recent developments in corresponding analysis methods are quite promising (for example the microassay technology), such observations are still laborious and hence, time and cost consuming.

In order to uncover traits with high potential for crop breeding, association studies with fruit metabolic QTLs and QTLs that modify whole-plant yield-associated traits have been applied (Schauer, Nat Biotechnol 24, 447-454 (2006)).

Thus, the technical problem underlying the present invention is the provision of reliable means and methods to assess the expression or potential for expression of a trait of a plant in an easily applicable, but highly reliable manner.

The technical problem is solved by provision of the embodiments characterized in the claims.

Accordingly, the present invention relates in a first aspect to a method for determining the correlation between the metabolite profiles (MPs) and the expression or potential for expression of a trait of a group of plants comprising the steps of

(a) determining the expression or potential for expression of said trait in plants of said group of plants, wherein said plants differ in their expression or their potential for expression of said trait;
(b) determining the MPs of said plants; and
(c) performing a correlation analysis between said determined expression or potential for expression of said trait and said determined MPs.

In a second aspect, the present invention relates to a method for determining or predicting the expression of a trait of a plant comprising the steps of:

(a) determining the MP of said plant;
(b) evaluating said MP based on the correlation between the MPs and the expression or potential for expression of said trait as determined by the above described method according to the first aspects of the invention; and
(c) deducing from said evaluation of (b), the expression or potential for expression of said trait exhibited by said plant.

The present invention solves the above identified technical problem since, as documented herein below and in the appended examples, it was surprisingly found that in sharp contrast to the fact that no strong correlation was observed between individual metabolites and one prominent trait of plants, namely biomass production/growth, a high correlation between biomass production/growth and metabolite profiles/specific combinations of metabolites was obtained. These surprising findings demonstrate the high predictive power of the metabolic composition/metabolite profile for the expression or potential for expression of a certain trait, like, e.g., biomass production/growth rate, and, therefore, offer the advantageous possibility to derive the expression or the potential for expression of a plant's trait. Based on this possibility, the present invention for the first time provides reliably means and methods for determining and predicting the expression of a certain trait of a plant based on the evaluation of the metabolite profile of said plant.

In the context of the present invention, the proof of principle that traits of plants, particularly complex traits, can be described by biomarkers, particularly complex biomarkers, like the metabolic signature/the metabolite profile, has been demonstrated by performing correlation analyses between metabolite profiles and one prominent trait of plants, namely biomass production/growth rate.

In order to make this proof, metabolic analysis of a recombinant inbred line (RIL) population of *Arabidopsis thaliana* showing segregation of a wide range of growth and the subsequent observation of the relationships of metabolite levels and growth characteristics of those genetically closely related individuals/lines has been performed in the context of the present invention. Moreover, quantitative trait loci (QTL) that are responsible for the observed variation have been identified herein.

A comprehensive analysis of the characteristics of the metabolic system of plants exhibiting different growth rates have been performed in the context of the present invention. Thereby, advantage has been taken of the metabolic profiling technology, (Fiehn, Nat Biotechnol 18, 1157-1161 (2000)) and recent improvements of the analytical procedures involving GC/ToF-MS (Fiehn, *Humana Press* Totowa MJ, (2006)) and of the data analysis (Lisec, Nature Protocols, In Press (2006)). For these purposes, the application of large segregating populations of plants, such as RIL populations, was of particular relevance, since the expression of a certain trait, particularly a complex trait, of multicellular organisms is usually governed by many genes that each contribute a small portion to the overall trait. In case of growth, as an example of such a (complex) trait, this determination by many genes has been shown for ,*e.g.*, mouse (Rocha Mamm Genome 15, 83-99 (2004)), chicken (Sewalem, Poultry Sci 81, 1775-1781 (2002)), *Arabidopsis* (El-Lithy, Plant Physio 135, 444-458)) or rice (Li, Planta Sci 170, 911-917 (2006)).

The present invention is based on the findings of the experiments described in the appended examples. These examples particularly show an integrated analysis of an *Arabidopsis thaliana* RIL population for vegetative biomass accumulation and metabolite profiles using the concept of genetical genomics (Jansen, Trends Genet 17, 388-391 (2001)). The RILs were derived from a cross between the *Arabidopsis thaliana* lines Col-0 and C24 (Törjék, Theoretical & Applied Genetics (2006)), which in previous studies showed strong transgressive segregation for biomass (Meyer, Plant Physio 134, 1813-1823 (2004)). The metabolic composition was analyzed by GC-MS based methods. In addition to this, six biomass quantitative trait loci (QTL) and 228 non-randomly distributed metabolite QTL were detected. It was shown that the biomass QTL coincide with multiple metabolite QTL, indicative of relations between variation in growth and changes in metabolite levels.

The use of a RIL population for an exploratory analysis of possible relations between growth and metabolite levels is particularly advantageous (over, *e.g.*, using environmental perturbations to modulate growth and metabolism) as it offers the opportunity to readily identify the genetic determinants of all studied traits as a means to unravel causal relationships in addition to the determination of correlations. Using the obtained data on growth and metabolite composition, two complementary approaches to investigate relationships between variation in growth and differences in metabolite levels were followed in the context of the present invention and are described in the non-limiting appended examples: i) pairwise and canonical correlation analyses were performed between biomass and metabolite values; and, moreover, ii) QTL mapping was carried out for all traits (shoot dry matter and 181 metabolite concentrations) and their co-localization pattern was investigated.

In the prior art, attempts were made to identify individual/simple biomarkers, like individual transcripts/individual metabolites in order to describe certain traits, also more complex traits like biomass production/growth.

Most of these attempts had no satisfactory outcome, since, as described above, particularly complex traits usually are related to a more complex network of biomarkers all partially representing such complex traits.

In contrast, in the context of the present invention, it has been found out that there are no individual/simple biomarkers like, for example, "magic compounds", which alone satisfactorily can describe the expression or potential for expression of a complex trait, like, e.g., biomass production/growth. It was one major merit of this invention to successfully demonstrate that rather a combination of many/several metabolites correlate to such a trait. In other words, it was found that the overall composition of the "metabolite profile" represents a complex trait of a plant, such as biomass production/growth rate.

As mentioned, the present invention provides an experimental proof that the overall metabolic composition in a plant is highly correlated with biomass, a finding which is of true fundamental importance for understanding plant metabolism and growth as well as for breeding strategies. As mentioned above, the important finding is that a combination of the levels of a large number of metabolites (canonical correlation: 0.73; p: $<10^{-64}$; see appended examples) rather than individual metabolites (pairwise correlation: <0.24; see appended examples) show a close relationship with growth. This indicates that variation in growth coincides with characteristic joint changes of metabolite levels, while individual metabolites may fluctuate independently of alterations in growth.

Accordingly, the present invention for the first time provides the above described direct proof that the overall metabolic composition is highly related to the expression of a certain trait of a plant, like, for example, biomass production and thus growth. The observations made herein further extend these findings towards the notion that major global changes in metabolism are the result of variation of a certain trait, like growth, rather than *vice versa.*

The findings provided herein are of immediate potential for a number of applied purposes. For example, the possibility to determine and predict the expression of certain traits of a plant on the basis of the metabolic signature of said plant revolutionizes the selection and thus breeding processes of plants. Particularly with respect to biomass producers such as trees that are cultivated for many years or even decades before harvest, the means and methods of the present invention are highly advantageous. The identification of certain plants that express (a) certain trait(s) in a desired manner, for example potentially high biomass producers, already at an early growth stage can result in enormous time and cost-savings, for example in selecting and breeding procedures. This is particularly of high relevance in the light of reduced availability of fossil fuels and increasing reliance on bio-derived energy. In this context, the importance of the means and methods of the present invention can hardly be overestimated.

It is a further advantageous property of the present invention, that the provided methods can be generalized to plants belonging to different groups of plants.

As also further described herein below, this means that there is no need that the plants for which the expression of a desired trait is to be determined or predicted are of the same group as the plants for which the correlation analyses between the MPs and the expression or potential for expression of said trait have been determined. This possibility for generalization provides a further potential for obviating time and cost consuming procedures, e.g. during plant breeding procedures.

A further advantage of the present invention is the possibility to particularly describe complex traits of plants, i.e. traits, the expression of which is determined by a network of genetic factors (and/or influenced by environmental factors), in an easy and reliable manner. This is realized by taking advantage of a biomarker that also reflects this complexity, namely the MP of a plant. As described above, biomass production is one example of such complex trait. It is determined by the expression of several genes (and/or influenced by several environmental factors, e.g. light, water, and/or nitrogen supply).

In contrast to state of the art approaches, where individual/simple biomarkers (like, e.g., single genes, single transcripts or individual metabolites) were tried to be used to also describe complex traits, the present invention provides means and methods that take advantage of a complex biomarker, namely the MP of a plant, that features the potential to reflect the complexity of a complex trait in its entirety.

In summary, the herein demonstrated high predictive power of metabolic composition for the expression or potential for expression of desired traits (like, e.g., biomass) opens new opportunities to enhance plant breeding. Thus, the present invention, inter alia, belongs to the field of metabolic profiling or metabolome analyses.

In the context of the present invention the term "metabolite profile" ("MP") means a set of metabolites together with the amount of each of the metabolites being comprised in said set of metabolites.

In order to obtain an "MP", metabolites in a biological sample are determined, qualitatively and quantitatively. In this context, the term "qualitatively determined" refers to the identification of a certain metabolite in the sample and the term "quantitatively determined" refers to the determination in the sample of the relative or absolute amount of each identified metabolite or each metabolite to be analyzed. The term "determining" an MP/the MPs as used herein is to be understood accordingly. Accordingly, the data resulting from a determination of (an) MP(s) to be performed in the context of this invention comprise (unique) mass intensity values for each metabolite comprised in the MP(s).

It is intended that an MP to be determined herein pertains to the respective biological sample, and hence, to the plant, the example is derived from. Common metabolite profiling approaches, and hence approaches for determining an MP/the MPs belong to the prior art.

In this context "identification of a certain metabolite" means finding out which particular (already known) metabolite is concerned. Moreover, this term is envisaged to also refer to the specification of (an) unknown metabolite(s) by a characteristic feature, like, for example, the retention time resulting from gas chromatography. As mentioned herein below, such (an) "qualitatively determined" unknown metabolite(s) can also contribute to the information deduced from a MP in context of the present invention.

As also mentioned above, the term "amount" of a metabolite as used herein means the relative or absolute amount of a metabolite in a biological sample to be observed. How such a metabolite can be indicated and its "amount" can be measured is known in the art, but also exemplarily described herein.

For example, the relative amount of a metabolite is indicated as a value of percentage, e.g. per cent of the amount of one, several or all (other) metabolites of the corresponding MP. For example, the absolute amount of a metabolite is indicated as a value of concentration, e.g. concentration in the tested sample, or absolute quantity of the metabolite, e.g. per tested sample.

As a non-limiting example, the amount of a metabolite comprised in an MP to be employed in the context of the present invention, is derived from the area of a peak corresponding to said metabolite and being obtained from, for example, chromatography, like gas chromatography.

The skilled person is readily in the position to find out which particular kind of MPs are suitable to be employed in the context of the present invention. For example, such kind of MPs are described in Fiehn (Nature Biotechnology 18, 1157 - 1161 (2000)).

It is preferred in the context of the present invention that in the MP(s) to be employed, the number of the therein comprised metabolites is as high as possible, but is at least as high as necessary.

In this context, the term "as high as possible" means that it is preferred that said MP comprises data for as many metabolites as can be observed by the corresponding measurement method(s). As described and disclosed herein, it is desired for the MPs to be employed to reflect the complexity of the trait to be observed. In other words, the more "complex" said MP is, i.e. the more metabolites it comprises, the more complex a trait to be successfully observed can be or the more safely and reliably a trait of a given complexity can be observed. Accordingly, in this context, the term "as high as necessary" means that said MP comprises data for as many metabolites as necessary in order to be able to safely and reliably determine a correlation between such MPs and the expression or potential for expression of a desired trait by employing the methods of the present invention.

The number and selection of metabolites identified/analyzed in the methods of the invention in order to determine an "MP" generally depends on the goal to be achieved by carrying out the methods of the invention, e.g. the trait to be observed, as well as whether (a) certain metabolite(s) is (are) present in the sample at all. It is typical for metabolic profiling as employed in the methods of the present invention to aim at quantitatively determining an as large as possible set of metabolites in order to obtain as much as possible metabolite data. This means that in the context of the present invention, it is preferred that the number of metabolites determined to obtain an "MP" is high. "High" in this context means that the number of metabolites is at least 5, at least 10, at least 20, at least 50, at least 100, at least 200, at least 500 or at least 1000, wherein the higher numbers of metabolites are preferred.

Although it is preferred that the number of metabolites to be determined in the context of the present invention is high, it is also envisaged that a relatively low number of metabolites can be comprised in an MP in the context of present invention. Particularly, when metabolites like the herein defined "main drivers of correlation" are determined, the methods provided herein can also be successfully applied, when the MP comprises only few of these "main drivers". In this context "few" metabolites or "a relatively low number of metabolites" means at least 3, at least 5, at least 7, at least 10, at least 15 or at least 20 metabolites, wherein the higher numbers are again preferred.

However, as also demonstrated in the appended non-limiting examples, in a preferred embodiment of the method of the invention, at least 20, more preferably at least 50, still more preferably at least 100, even more preferably at least 150 and most preferably at least 200 or even at least 300 metabolites are quantitatively determined and hence, are comprised in a preferred MP as employed in the context of the present invention.

The term "metabolite", in the context of the present invention, refers to any substance within a plant cell or produced by a plant cell, including secreted substances, which can be quantitatively determined, for example, by applying corresponding methods known in the art, e.g. methods that can resolve mass differences between metabolites. As used herein, the term "metabolite" implies one of its own definitions, namely being a compound of the "metabolism" of a plant. It is known in the art that "metabolism" comprises "anabolism" and "catabolism" of a plant. Accordingly, in a preferred embodiment, the "metabolites" in the context of this invention are metabolites of the plant's catabolism and/or anabolism. The term "Metabolite(s)" in the context of this invention does not refer to macromolecules (i.e. biopolymers) such as DNA, RNA or proteins. Particularly preferred are metabolites with a low molecular weight. Preferably the metabolites have a molecular weight of not more than 4000 Da, preferably not more than 2000 Da, more preferably not more than 1000 Da. Typically, the metabolites to be analyzed may belong to the following, non-limiting list of compounds: carbohydrates (e.g. sugars, oligo- and polysaccharides such as polyglucans as for example starch or polyfructans), sugar alcohols, amines, polyamines, amino alcohols, aliphatics, aliphatic alcohols, amino acids, lipids, fatty acids, fatty alcohols, organic acids, organic phosphates, organic ions, other inorganic ions bound to metabolites, nucleosides, nucleotides, sugar nucleotides, purines, pyrimidines, such as adenine and uracil, sterols, terpenes, terpenoids, flavons and flavonoids, glucosides, carotenes, carotenoids, cofactors, ascorbate, tocopherol, vitamins, polyols, organic amines and amides such as ethanol amine and urea and/or heterocyclic compounds such as nicotinic acid.

As is evident from the appended Examples, the methods of the invention also involve analyzing metabolites of which the chemical nature is unknown. However, metabolites of unknown chemical nature may nevertheless provide informative data on the biological sample analyzed, and hence, the MP pertaining to it. For example, such informative data may be the retention time in, e. g. resulted from gas chromatography, and/or the amount of a metabolite of unknown chemical nature (for example deduced from the area of a peak resulting from gas chromarography), as well as the absolute numerical value of the COR and its algebraic sign. It is clear that, if a metabolite of unknown chemical nature is revealed by carrying out the methods of the invention to have an interesting property or characteristic behavior, this metabolite may be further characterized by applying suitable analytical methods known in the art. For example such methods take advantage of a comparison of the retention time of said metabolite of unknown chemical nature resulting from gas chromatography with the retention times of corresponding known standards.

In a preferred embodiment, in order to obtain an MP to be employed in the context of the present invention, metabolites comprising sugars, sugar alcohols, organic acids, amino acids, fatty acids, vitamins, sterols, organic phosphates, polyamines, polyols, nucleosides, purines, pyrimidines, adenine, uracil, organic amines and amides such as ethanol amine and urea and/or heterocyclic compounds such as nicotinic acid are envisaged to be analyzed.

In a more preferred embodiment, particularly when the trait to be observed is biomass production, the MP(s) to be employed in the context of the present invention comprise(s) metabolites of the central carbon or nitrogen metabolism, metabolites of membrane and/or (phospho)lipid biosynthesis, metabolites of nitrogen assimilation, metabolites of stress response, metabolites acting as plant hormones, metabolites acting as regulating factors such as co-factors, metabolites acting as signals (like messengers, for example second messengers) and/or metabolites of the secondary metabolism of plants.

In this context, examples for metabolites of the central carbon metabolism are sugar phosphates like glucose-6-phosphate and fructose-6-phosphate, members of the TCA cycle such as succinate and citrate, malate and sucrose. An example for a metabolite of the central nitrogen metabolism and/or the nitrogen assimilation is glutamine. Examples for metabolites of the membrane and/or (phospho)lipid biosynthesis are glycerol-3-phosphate, ethanolamine or sinapine. Examples for metabolites of the stress response are polyamines such as putrescine, spermidine and ornithine, nicotinic acid and trehalose. Examples for metabolites acting as signals (like messengers, for example second messengers) and examples for metabolites of the secondary metabolism of plants are known in the art. An examples for a metabolites acting as a plant hormone or as a regulating factor is ascorbic acid (vitamin C).

For example, the MP(s) to be employed herein comprise(s) metabolites selected from the group consisting of: ethanolamine; fructose-6-phosphate; citric acid; glutamine; glycerol-3-phosphate; sinapic acid (cis); raffinose; ornithine, putrescine; glucose-6-phosphate; spermidine (major); sinapic acid (trans); sucrose; citramalic acid; ascorbic acid; tyrosine; succinic acid; malic acid; trehalose; nicotinic acid; maleic acid, phenylalanine; and salicylic acid. With respect to these examples of metabolites, the preceding ones are preferred to be employed herein.

These and further examples of metabolites to be employed in the context of the present invention are given in Table 1, herein-below. In Table 1, those metabolites are preferred, the COR of which is high. It is generally preferred herein that the metabolites to be employed have high COR values, which, for example result from a correlation analysis between the expression of a trait and MPs of plants.

It is clear for a skilled person that the above mentioned groups of metabolites or particular metabolites are not limiting and that the particular metabolites being comprised in an MP may vary, e.g. dependent on the sample from which they are derived and/or dependent on the particular trait to be observed.

The term "biological sample", from which the metabolites to be determined in order to obtain an MP are derived, encompasses any amount of material comprising plant cells, tissue or organ or being derived from plant cells, tissue or organ that is susceptible to the methods as disclosed herein. In the present context, the term "plant cell" refers to any conceivable plant entity comprising or secreting the metabolites to be determined in the context of the present invention. Accordingly, the methods of the present invention may take advantage of any type of plant cell, e.g. wild-type or transformed, transduced or fused cells, or derivatives thereof such as membrane preparations, liposomes and the like. The cells may furthermore be part of a tissue, an organ or a complete plant. The cells may be in a naturally occurring form or in a man-made form such as in a cultured form, e.g. cell culture, protoplast culture, tissue culture or the like.

For example, the metabolites to be determined in the context of the methods of the present invention may be derived from a "biological sample" that is derived from a whole plant or a part or an organ of a plant. In this context, a "part" of a plant may be (a) root(s), the shoot, (a) branch(es), (a) leave(s), (a) fruit(s), the florescence and the like. An "organ" of a plant may be a root, the shoot, a branch, a leave, the hypocotyl, a bud, a flower, a fruit and the like. It is preferred that the herein employed "biological sample" is derived from (a) leave(s). These examples are not limiting and a skilled person is able to obtain any "biological sample" suitable to be employed in the context of the present invention.

It is preferred in the context of the present invention that a step of deriving the "biological sample", for which the metabolites are to be determined in the context of the present invention, is as little invasive as possible. This means that the plant to be tested is disturbed as little as possible in its development, when applying the methods of the present invention. This is particularly relevant for those methods disclosed herein that refer to the determination or prediction of the expression of a trait to be observed. For example such methods are the methods for breeding of a plant as disclosed herein. Accordingly, the "biological sample" is preferably of such part or organ of a plant, which is not crucial for the (unimpaired) development of said plant. Therefore, non-limiting examples for such a part or organ may be a leave (e.g. a cotyledone), a bud, a flower, a branch, a fruit, and the like.

The term "deriving" used in connection with characterizing the "biological sample" means any kind of measures a skilled person may apply in order to modify the plant cells, tissue or organ or the direct environment of the plant cells, tissue or organ, wherein the "direct environment" is characterized by the presence of at least one metabolite produced by the cells, in order to prepare a sample for use in quantitatively determining the metabolites contained therein in order to obtain an MP to be employed in the methods of the invention. Such measures may for example involve typical sample preparation or extraction techniques common to those skilled in the art. The direct environment may for example be the extracellular space around a cell, the apoplast, the cell wall or the culture medium. Furthermore, the biological sample derived from a cell may be a certain part of the cell, for example certain cellular compartments such as plastids, mitochondria, the nucleus, vacuoles etc.

A "biological sample" in the context of the present invention can for instance be fresh material such as a tissue explant, an exudated fluid or an aliquot from a cell culture, preferably deprived of the culture medium, that may be directly subjected to extraction. On the other hand, "biological samples" may also be stored for a certain time period, preferably in a form that prevents degradation of the metabolites in the sample. For this purpose, the sample may be frozen, for instance in liquid nitrogen, or lyophilized.

The samples may be prepared according to methods known to the person skilled in the art and as described in the literature. In particular, the preparation should be carried out in a way suitable to the respective method of the present invention to be applied. Furthermore, care should be taken that the respective compounds to be analyzed are not degraded during the extraction process. Biological samples for metabolite analyses may for example be prepared according to procedures described in Roessner (Plant J. 23 (2000), 131-142).

Furthermore, the biological sample to be employed in the context of the present invention may optionally be fractionated and/or purified so that the sample contains a subset of the metabolites contained in the sample.

By this fractionation and/or purification step, it is for example possible to select low abundant metabolites out of the whole pool of metabolites whereby, without this step, these metabolites might not be detectable for example because their signals are superimposed with strong signals of highly abundant metabolites.

The fractionation and/or purification may be carried out according to standard procedures known in the art. It is clear that preferably procedures should be used that do not or at least only to a low tolerable degree change the distribution of the metabolites in the sample.

The determination of metabolites of a biological sample, to be performed in order to determine an MP, may be carried out by any known suitable method, for example by a method that can resolve mass differences between different metabolites. This may involve various nuclear magnet resonance (NMR) and mass spectrometry (MS) techniques that are known to a person skilled in the art, whereby mass spectrometry is preferred in the context of the present invention. Different suitable NMR and MS techniques are for instance described in Wittmann (Adv. Biochem. Engin. Biotechnol.

74 (2002), 39-64) and Szyperski (Q. Rev. Biophys. 31 (1998), 41-106). Preferred setups for MS techniques for use in the present invention involve the combination of MS with gas chromatography (GC) as it is routinely used in state-of-the-art metabolite analyses, such as GC-MS described in the appended Examples and the corresponding literature to which the examples refer with respect to this matter.

In cases of ambiguous fragment interpretation, analyses using GC-MS-MS or corresponding MS tandem arrangements may support the identification of isotopomer fragment pairs. For example, GC-MS systems supplied with ion trap technology allow the selection of individual primary fragments and subsequent secondary mass spectral fragmentation (Birkemeyer, J. Chromotography A 993 (2003), 929-937; Mueller, Planta 216 (2002), 44-56. These MS-MS mass spectral fingerprints may allow an unequivocal identification of corresponding primary ions.

As mentioned, the determination of the amount of metabolites of interest can be done according to well-known techniques known in the prior art. Preferably, techniques are applied that allow the identification and quantification in one step and, advantageously, are suited to record the respective metabolites contained in the sample in a comprehensive manner.

Further methods for quantitatively determining the metabolites for use in accordance in the present invention include liquid chromatography/mass spectrometry (LC/MS), the use of radioactivity in connection with suitable methods known to the skilled person, thin layer chromatography (TLC), capillary electrophoresis (CE), direct injection MS, flow injection MS, MS/MS, MS/MS/MS, and further combinations of MS steps (sequential MS techniques: MSn techniques), fourier transform ion mass spectrometry (FT/MS), gel permeation chromatography (GPC), TLC, CE, HPLC, GPC, any other chromatographic or electrophoretic technique or any mass spectrometric technique which is hyphenated in-line or off-line to mass spectrometry. If appropriate, any of the above methods may be combined.

An exemplary non-biased analyses is described in Fiehn (Anal.Chem. 72 (2000), 3573-3580). In this study, of different plant mutants, 326 distinct compounds (ranging from primary polar metabolites to sterols) were detected and relatively quantified, including both identified and non-identified compounds, by applying GC/MS analyses. Another example of a GC/MS analyses that can be applied in the method of the invention has been described by Roessner (Plant Cell 13 (2001), 11-29), where it was used for comprehensively studying the metabolism in potato tubers.

It is also envisaged in the context of the present invention that the metabolites to be detected are chromatographically separated prior to their quantitative determination.

This embodiment refers to the chromatographic separation which has already been described above by referring to the particularly preferred example of using gas chromatography in settings such as GC-MS or GC-MS-MS. Other suitable chromatography methods such as HPLC, RP-HPLC, ion-exchange HPLC, GPC, capillary electrophoresis, electrophoresis, TLC, chip-base micro-fluidic separation, affinity-interaction chromatography using antibodies or other ligand-specific binding domains may also be used in this regard.

In another embodiment, it is envisaged that the methods of the invention further comprise a step of introducing external standards for one or more of the quantitatively determined metabolites.

The introduction of external standards or standard dilution series allows the determination of metabolite concentrations in absolute terms. By contrast, embodiments of the method of the invention in which no external standards or standard dilution series are applied allow the exact quantification in relative terms, i.e. concentration changes observed relative to reference quantities as observed in experimental control samples. The introduction of external standards and the provision of such standards may be carried out as described in the literature and as is known by the person skilled in the art.

As has been mentioned above, the methods of the invention also include the quantitative determination of metabolites the chemical nature of which is yet unknown. Accordingly, it is also envisaged that the herein employed determination of an MP further comprises the step of identifying one or more of the unknown metabolites which are quantitatively determined.

This identification may be carried out by analytical methods known to the skilled practitioner and described in the literature.

In a particularly preferred embodiment, this identification comprises identification by secondary fragmentation.

Secondary fragmentation techniques may be carried out by methods known in the prior art, in particular by GC-MS-MS or other sequential MS techniques (MSn techniques). Separate recording and subsequent comparison of chemical intermediates from MS-MS fragmentation pathways of, e.g., $^{13}$C isotopomer pairs is highly facilitated by providing the number of carbon atoms present within each observed MS-MS fragment.

In an especially preferred form of this embodiment, identification of the metabolites comprises electron impact ionisation, MS-MS technology and/or post source decay analyses of molecular ions or fragments.

Such techniques are known to a person skilled in the art.

The term "(potential for) expression of a trait" is used herein in the same meaning as the terms "expression or potential for expression of a trait" or "expression of a trait or potential for expression of a trait".

In the context of the present invention, the term "trait" means any kind of a feature or character of a plant, particularly any kind of a feature or character of a plant, the (potential for) expression of which can be detected, and whichis, at least in part, genetically determined. "Genetically determined" in this context means that the degree of expression of at least one gene, preferably of a set of genes, determines, at least in part, said feature or character.

Without being bound by theory, the MPs to be employed herein, for example as complex biomarkers, offer the possibility to reliably describe complex traits. Accordingly, in a preferred embodiment of the present invention, the "trait" to be observed is a complex trait. "Complex trait" in this context means that a trait is determined not only by one or a few, but by several genes. For example, such "complex trait" is determined by more than 1, preferably more than 2, preferably more than 3, preferably more than 5, preferably more than 7, preferably more than 10 genes, preferably more than 50 genes, preferably more than 100 genes and more preferably more than 300 genes.

One particular, non-limiting example of such a "complex trait" of plants is biomass production/growth. As also demonstrated in the appended examples and without being bound by theory, this complex trait is determined by at least more than 5, particularly by at least 6 genes, which can be deduced from the findings that 6 quantitative trait loci (QTLs) for biomass production/growth were detected (see Example 4).

However, even though the methods provided herein are particularly advantageous to observe complex traits, they are not limited to such traits and can also be applied to "simple traits", i.e. traits which are determined by only a few genes or only one gene. "Few" in this context, for example may be less than 6, 5, 4 or less than 3 genes.

(A) particular "trait(s)" to be observed in the context of the present invention may, for example, be of morphological nature, anatomical nature, physiological nature, ecophysiological nature, pathophysiological nature, and/or ecological nature, and the like.

For example, "traits" of morphological nature may be size, weight, number, and the like, of roots (like, e. g., storing roots), of shoots, like side shoots (like, e. g. storing shoots), of leaves (like, e. g., (succulent) storing leaves), of flowers or florescences, of fruits, of seeds (like, e. g., grains), and the like. Further examples of "traits" of morphological nature may be size, height, weight, and the like, of the whole plant.

"Traits" of anatomical nature, for example, may be the anatomical structure of vascular bundles (like, for example, development of the crown syndrome), of the medulla, of the wood or of other tissues, and the like.

"Traits" of physiological nature, for example, may be contents of compounds, in particular storage compounds, like lignin, cellulose, starch or sugars (or other nutrients like fats or proteins), fibers, water, vitamins or compounds of the secondary metabolism of plants, fertility, and the like.

"Traits" of ecophysiological nature, for example, may be tolerance or resistance against environmental influences (including "man made" environmental influences) like drought, heat, cold, hypoxia and/or heavy metals, and the like.

"Traits" of pathophysiological nature, for example, may be tolerance or resistance against pathogens like viruses, fungi, bacteria and/or nematodes, and the like. However, also the susceptibility to, for example, these pathogens may be a "trait" in the context of the present invention.

"Traits" of ecological nature, for example, may be the potential for attraction or repellence to phytophages or nectar/pollen-collecting animals (like insects), the capacity to adapt to changes in the environment, and the like.

It is of particular note that a given "trait" in the context of the present invention may not belong to only a single one of the above mentioned categories, but also to several of them, and, furthermore, to other categories not explicitly mentioned herein.

The herein mentioned categories of traits, as well as the herein mentioned examples of traits are by far not limiting. Further "traits" of plants, e. g. in the form of detectable features or caracters, are well known in the art. The person skilled in the art is readily in the position to figure out further "traits", particularly of traits, the observation of which is economically desired, based on his common general knowledge and the disclosure in the prior art. The above mentioned and also further "traits" being observable in the context of the present invention can particularly be deduced from corresponding pertinent literature.

A further example of a "trait" in the context of the present invention is the flowering time of a plant.

A particularly preferred "trait" in the context of the present invention is wood property (like, e. g., wood stability, wood resistance against rotting and the like) or wood composition (like, e. g. cellulose and/or lignin content and the like), and the like.

In the context of the present invention, the term "expression of a trait" refers to how a trait is expressed in terms of measurable parameters. For example, in case the "trait" to be observed is biomass production/growth, said parameters, for example, are volume/mass expansion per time or volume/mass at a certain point in time. In this context, "mass" can mean dry weight or fresh weight of (a) plant(s) to be employed.

Further non-limiting examples of measurable parameters in this context are number, amount, concentration, length, density, area, flexibility and the like.

With respect to the methods for determining the correlation between the MPs and the (potential for) expression of a trait as disclosed herein, the term "determining the (potential for) expression of said trait" means that the above-mentioned measurable parameters corresponding to said trait are measured. It is preferred, that this measurement takes advantage of suitable measurement approaches. Such approaches are well known in the art. For example, such approaches are weighing, counting, sizing, detecting the colour, and the like. The certain measurement approach to be chosen depends on the (potential for) expression of the trait to be observed, and hence, to the corresponding measurable parameter(s). The skilled person is readily in the position to find out which certain measurement approach is "suitable" for a certain trait to be observed.

In the methods for determining the correlation between the MPs and the (potential for) expression of a trait of a group of plants as provided and described herein, plants should be employed which differ in their (potential for) expression of said trait. This ensures that, a correlation with the corresponding MPs of said plants, each reflecting a certain (potential for) expression of said trait, can be determined.

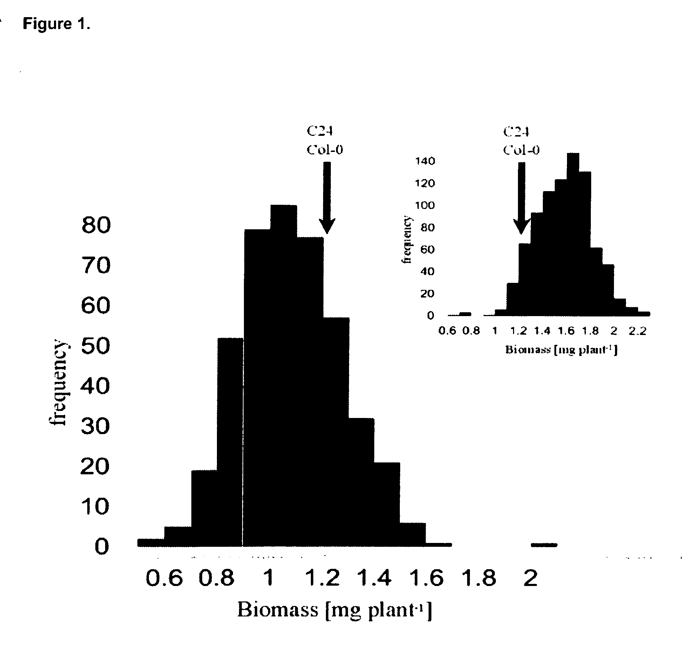

The term "plants [that] differ in their (potential for) expression of [a] trait" as used herein means that different individual plants of a group of plants as defined herein exhibit different (potentials for) expression of a trait. Particularly, this means that the (potential for) expression of a trait of at least one plant of said group of plants is reduced or enhanced compared to a certain standard, like, for example, the (potential for) expression of the said trait of at least one other plant of said group of plants or the averaged (potential for) expression of said trait of a certain number of plants of said group of plants. For example, as demonstrated in the appended non-limiting examples, the individuals of the *A. th* RIL population as employed herein exhibit a range of different biomass production among each other (for example as depicted in mg per plant at the time of harvest; see Fig. 1), following a relatively equal distribution. Said *A. th* RIL population is a non-limiting example for a group of plants to be employed in the context of the present invention.

It is preferred that the expressions of a trait to be observed or the potentials for expression of a trait to be observed of the different plants of a group of plants to be employed herein exhibit a wide range and/or show a relatively equal distribution within this/a range.

Without being bound by theory, such a wide range and/or equal distribution may result in particularly highly reliable outcomes of the analyses of the correlation between the MPs and the (potential for) expression of a trait as disclosed herein.

An expression that can be detected of a trait to be observed herein, may for example be visually identifiable, such as a morphological (or anatomical) outcome.

Furthermore, such expression of a trait may, for example, also be non-visually identifiable, such as a physiological (or anatomical) outcome, like an outcome of the chemical composition of certain compartments of a plant or a plant cell (like, e.g., cell wall, cytosol, membrane systems (like the endoplaspatic reticulum) or lumens enclosed therein (like the intrathylacoid lumen or the grana matrix of chloroplasts), and the like.

Particularly for non-visually identifiable expressions of a trait or potentials for expression of a trait, an application of the means and methods provided and described herein is particularly advantageous.

The "(potential for) expression of a trait" may be influenced by environmental factors. For example, such factors are light supply, water supply, nitrogen supply, soil composition, and the like.

Thus, the "expression of a trait" on the one hand may be a function of, i.e. determined by, the genetic background of a trait (the gene(s) that determines the trait), and on the other hand a function of the environmental impact on the expression of said genes, and hence on the expression of the trait.

Accordingly, without being bound by theory, an MP that represents a certain (potential for) expression of a trait may reflect both, the genetic background of said trait and the environmental impact on (the potential for) its expression.

In a preferred embodiment of the present invention, it is particularly desired for the herein provided methods of the present invention that the (potential for) expression of a trait that differs between plants to be tested/observed, reflects differences in the genetic background of said plants.

For example, when employing the herein provided methods of determining or predicting the expression of a trait of a plant, the corresponding methods of breeding of a plant and/or the method of screening for a plant, it may be, inter alia, desired that differences in the (potential for) expression of the plants to be tested are only due to differences in the genetic background of said plants.

Accordingly, for such applications, it is particularly preferred that the plants to be tested are all grown under the same environmental conditions, in order to be able to identify those plants, the desired (potential for) expression of a trait of which is due to the genetic background of said plant. A non-limiting example for an application of the present invention in order to find out such plants, is given in the appended examples.

The most preferred "trait" to be observed by the methods of the present invention is biomass production/growth of a plant. Biomass production/growth is a complex trait in terms of the present invention, i.e. it is determined by several genes (Jompuk, J Exp Bot 56, 1153-1163 (2005), Schön, Genetics 167, 485-498 (2004), Hittalmani, Euphytica 125, 207-214 (2002), Wullschleger, Can J Forest Res 35, 1779-1789 (2005)).

As already mentioned above, this was also demonstrated in the appended examples (for example by the therein disclosed QTL analyses).

Accordingly, since it is preferred in the context of the present invention to observe complex traits, the methods of the present invention are particularly useful to observe biomass production/growth of a plant.

The meaning of the terms "biomass", "biomass production", "growth" and "growth rate", and the like, are known by the person skilled in the art. For example, it is known in the art that biomass production, and hence the resulting biomass, may, inter alia, be the result of the growth/growth rate of a plant.

It is known in the art that growth of a plant mainly encompasses elongation growth as well as primary and secondary thickening. These kinds of growth of a plant are basically volume growth. In view of this, in the context of the present invention, the term "biomass production/growth" or "biomass production/growth rate" is to be understood accordingly.

However, in the context of the present invention it is also envisaged that the term "biomass production/growth (rate)" also encompasses changes in a plant that contribute to biomass production/biomass of the plant, independently from its volume growth, e.g. changes that happen after volume growth has ended. For example, such changes are procedures of incorporation of compounds (e.g. lignin or cellulose, and the like) into the cell wall. Such procedures are known as ligninification or hardening of the cell wall. Moreover, examples for such changes are the formation of further layers of the cell wall, for example known as the formation of secondary and tertiary cell walls.

Accordingly, it is evident that not only volume growth (which is mainly due to an uptake of water into cells of a plant), but also "growth" in the form of the above mentioned "changes" contribute to biomass (production) of a plant. This is particularly relevant and/or evident in case biomass (production) of a plant is expressed in terms of dry-matter.

"Biomass production/growth" of a plant can be described by quantifiable parameters, like the growth rate of a plant or of a plant's tissue/organ.

In the art, "growth rate" is usually described in terms of volume or weight (for example dry weight) per time unit. "Biomass production"/"biomass" can, for example, also be expressed in terms of a distinct indication of mass (for example dry weight) at a given time, e.g. the point in time at harvest or at the conduction of a certain measurement. Such measurement can, for example, be a method, measurement or analysis as disclosed or described herein.

The meaning of the term "potential" as used herein, or in the more narrow sense the term "potential for expression of a trait", refers to a given status of a plant at a certain point in time that determines a future expression of said trait, i.e. an expression of said trait temporally after said certain point in time. Preferably "status" in this context means the given sum of genetically, morphologically, anatomically, physiologically etc. settings of a plant which contribute to the future phenotype of the plant. In other words, the term "potential (for expression of a trait)" means the potential of a plant at a certain point in time to express a trait temporally after said point in time. More simple, "potential for expression" means the "capacity of expression in the future".

The the term "predicting" or "predict" as used herein, or in the more narrow sense the term "predicting the expression of a trait", means that the future expression of a trait is anticipated. This anticipation is based on the potential for expression of said trait the plant exhibits at a certain point in time, e. g. the point in time when the methods of the present invention are applied. Thereby, said point in time is temporally earlier than a point in time corresponding to the "future" expression to be anticipated.

In view of the above, it is to be seen that the "predicting the expression of a trait" that can be performed according to the method provided herein at a certain point in time is based on the potential for said expression of said trait at said point in time. Accordingly, the term "deducing the potential for expression" as used herein means deducing the predicted expression of a trait, and hence, deducing its future expression.

It is of note that the meaning of the terms "predicting", "predict", "predicted" etc. as used in the context of the methods for "predicting" the expression of a trait (e. g. biomass production/ growth) as disclosed herein, differs from that of the terms "prediction", "predicted", "predictor variables" etc. as used in the context of the statistical methods described herein and to be employed in the context of the present invention. For example, in the latter context, the respective terms are used when a trait is "predicted" from a corresponding metabolite matrix, wherein said "prediction" is based on the so called "predictor variables". This "prediction" explicitly does not refer to an anticipation of future expression of a trait. A skilled person is readily in the position to distinguish between the different meanings of the afore-mentioned terms, based on the respective context in which they are used.

According to the present invention, if a correlation between (an) MP(s) and a expression or potential for expression of a trait of a group of plants is found at a certain point in time, the MP of a plant can be used to predict the expression of said trait also for the future development of said plant. Thereby, said plant generally may not be, but preferably is, of the same group of plants for which the correlation was found.

For the purpose of predicting the expression of a trait to be performed according to this invention, the future expression of said trait is deduced from the expression or potential for expression of said trait determined at a certain point in time.

The term "correlation" as used herein belongs to the field of statistics. The general meaning of the term "correlation" is well known in the art. In general, "correlation" is known to indicate the strength and direction of a relationship, in most cases a more or less linear relationship, between two (random) variables.

Applied to the present invention, the two (random) variables, to which the term "correlation" in the generally known sense refers, are, firstly, an MP and, secondly, the (potential for) expression of a trait. Accordingly, the term "correlation" as used herein refers to such kind of correlations that are capable to describe the relationship between a first simple variable, like the (potential for) expression of a trait, and a second composite variable, like an MP. "Simple" in this context means that a variable is a single variable that can be described by its corresponding parameter (for example weight, growth rate, number, size, and the like).

"Composite" in this context means that the variable comprises several "sub-variables" (like, e. g. several metabolites), each of which can be described by its corresponding parameter (for example amount or concentration). Accordingly, the term "correlation" as used herein also refers to such kind of correlations that are capable to describe the relationship between one single variable on the one hand (like the (potential for) expression of a trait) and (a) multiple variable(s), like an MP, on the other hand. Particularly, this means that the term "correlation" refers to a correlation between combinations of metabolites (i. e. their amonts/levels) comprised in the determined MPs and the (potential for) expression of a trait to be observed.

Commonly, and also in the context of the present invention, a "correlation" is depicted in the form of a corresponding "correlation coefficient", for example denoted and referred to herein as "COR". A correlation coefficient can further be depicted together with the so called "p-value". The meaning of the "p-value" is well known in the art and, for example, can be described as the probability of obtaining a result to appear or as the significance of a given result, e.g. a result of a correlation analysis, like the COR.
It is clear for the skilled person, and applies here mutatis mutandis, that in case maximum correlation occurs, COR is 1 and in case no correlation occurs, COR is 0. It is further known that the lower the p-value, the more significant are the corresponding results. It is further known in the art that for negative or positive correlations, the corresponding COR is given as a negative or positive value, respectively.

It is preferred that the absolute numerical value of the COR corresponding to the correlation to be determined according to the present invention is high. In the context of the present invention, a "high" absolute numerical value of the COR means a "strong" correlation, a "medium" absolute numerical value of the COR means a "moderate" or "medium" correlation and a "low" absolute numerical value of the COR means a "small", "weak" or "low" correlation. "High" in this context means at least 0.5, preferably at least 0.6, more preferably at least 0.7, more preferably at least 0.8 and most preferably at least 0.9. "Medium" means between 0.3 and 0.5, particularly between 0.35 and 0.45. "Low" means less than 0.3, particularly less than 0.2 and more particularly less than 0.1. In a preferred embodiment, for absolute numerical COR values less than 0.1, no correlation at all is assumed.

In view of the above, it is clear that the term "determining the correlation" as used herein refers to both, determining how an MP and the (potential for) expression of a trait correlate, but also if these two variables correlate at all. The herein disclosed methods of determining or predicting the expression of a trait and the method for identifying QTLs, as well as the methods depending thereon, are based on the outcome that these two variables indeed correlate. As mentioned above, the proof of principle that this is generally possible is provided herein.

As mentioned above, the COR corresponding to the correlation between (an) MP(s) and an (potential for) expression of a trait to be determined herein is preferably high. Accordingly, in a particularly preferred embodiment, it is assumed that (an) MP(s) and an (potential for) expression of a trait only correlate at all, when the absolute numerical value of the corresponding COR is at least 0.5, 0.6, 0.7, 0.8 or 0.9, wherein the higher values are preferred.

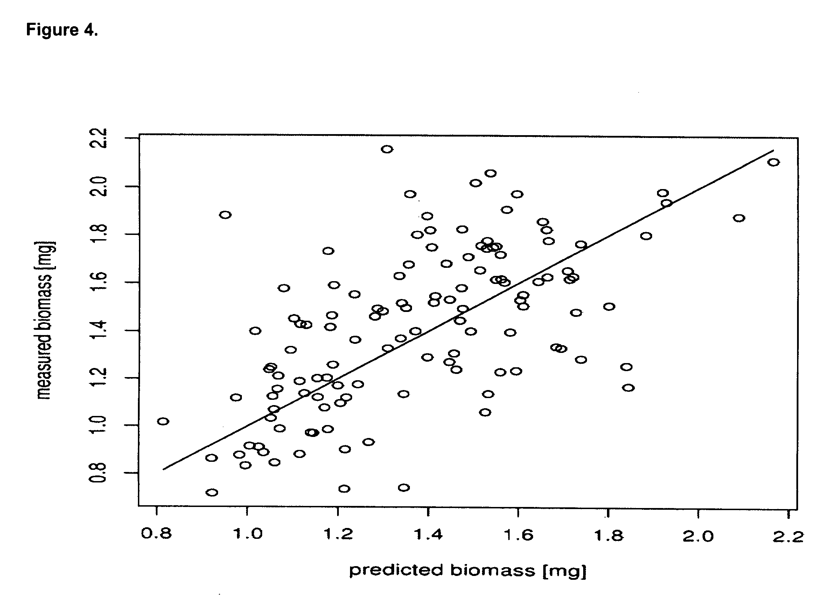

Particularly, in the context of the present invention, the term "correlation between the MPs and the (potential for) expression of a trait of a group of plants" means how the MPs of different plants of said group of plants, and particularly the amount of every single metabolite being comprised in said MPs, vary dependent on differences in the (potential for) expression of said trait among said plants. In other words, the correlation to be determined in the context of the present invention provides information on how and if MPs, and particularly the amount of every single metabolite being comprised in said MPs, differ among the plants of a group of plants dependent on a different (potential for) expression of a trait to be observed.
Accordingly, the methods for determining a correlation as disclosed herein, and the corresponding correlation analyses to be performed, respectively, result in the information for all of the single metabolites comprised in an MP, in which direction, i.e. increasing or decreasing, and to which extent, i.e. to which multiple, their amount differs, dependent on the (potential for) expression of the trait to be observed.
Moreover, the methods for determining a correlation as disclosed herein, and the corresponding correlation analyses to be performed, respectively, provide the information, whether an MP as a whole, and, accordingly, the whole set of metabolites comprised in said MP, depend on the (potential for) expression of the trait to be observed
Accordingly, the results of a method for determining a correlation as disclosed herein provides the information if and how differences in (the potential for) expression of a trait of (a) plant(s) are reflected by the differences in the MP(s) of said plant(s).
A non-limiting example for "determining a correlation between the MPs and the (potential for) expression of a trait of a group of plants" according to the invention is provided herein and is described in the appended examples. A non-limiting example of a result/outcome of such a determination, i. e. the information resulting out of it, is given in Figure 4 and the corresponding examples and Tables. For these examples, the trait to be observed exemplarily was biomass production/ growth. As demonstrated in the appended examples, the correlation between the (potential for) expression of this particular trait and the MPs of a group of plants (in the particularly demonstrated case an RIL population of *Arabidopsis thaliana* and an MPs comprising 181 single metabolites) for example can result in a canonical correlation of 0.73 by a corresponding p-value being $< 10^{-64}$.

The term "correlation analysis" as used herein refers to any (statistical) analysis approach suitable to obtain the "correlation" as defined herein. Accordingly, it is envisaged that the "correlation analysis" to be performed in the context of this invention is suitable to find out if and how the MPs and the (potential for) expression of a trait correlate. Since an MP comprises multiple single metabolites (which, as mentioned above, can be seen as multiple variables), a "correlation analysis" "suitable" to be employed herein is capable to determine a "correlation" between multiple variables (like multiple metabolites) on the one hand and a single variable (like the (potential for) expression a certain trait of a plant) on the other hand. Such "correlation analysis" comprise correspondingly applicable statistical methods. Such statistical methods should be capable to calculate the highest possible correlation between combinations of metabolites (i. e. their amounts/levels) extracted from the determined MPs and the (potential for) expression of a trait to be observed.

Based on his common general knowledge and the disclosure provided herein, the skilled person is readily in a position to find out correlation analysis methods, and hence, correspondingly applicable statistical methods, that are suitable to be employed in the context of the present invention.

Examples for such correlation analysis methods are described herein and are given in the appended examples.

The correlation analyses "suitable" to be employed herein particularly are correlation analyses that result in a mathematical function between (an) MP(s) and the expression of a trait. For example, such correlation analyses take advantage of a multiple regression method, i. e. of a multivariate analysis. For example, such a multiple regression method is a canonical correlation analysis (CCA), an ordinary least square (OLS) regression analysis, a partial least squares (PLS) regression analysis, a principal component regression (PCR) analysis, a ridge regression analysis or a least angle regression (LAR) analysis.

In a preferred embodiment of the present invention the "correlation analyses" take advantage of a multiple regression method further combined with a cross validation analysis.

A preferred correlation analysis to be performed in the context of the present invention takes advantage of CCA, preferably of a combination of CCA and PLS (particularly of CCA followed by PLS), and even more preferably of CCA followed by PLS combined with a cross validation analysis. Examples of such preferred correlation analyses to be employed in the context of the present invention are also described in the appended examples.

In a further particular embodiment of the present invention, the correlation analyses to be employed may additionally comprise the non-linear version of the above-described regression analyses or analyses that take advantage of supervised machine learning. Examples of such non-linear versions are non-linear versions based on kernel methods. Examples of analyses that take advantage of supervised machine learning are analyses relying on support vector machines (SVMs) or relevance vector machines (RVMs).

For example, when applying the "correlation analysis" to be employed herein, the multiple regression method, i. e. the multivariate analysis (e. g. the CCA), calculates the highest possible correlation between combinations of the metabolites of (an) MP(s) and the (potential for) expression of a trait.

Optionally, said multivariate analysis can be preceded by a pairwise correlation analysis taking advantage of a suitable linear model, resulting in pairwise correlations between every single metabolite comprised in the MP(s).

In this context, the skilled person is readily in the position to find out suitable linear models to be applied correspondingly. Such linear models, for example, may be the Pearson correlation, as described by the following formula (1):

$$(1) \qquad B = c_i x_i$$

An example for a more complex multiplicative model to be employed in the context of a multivariate analysis (e. g. the CCA) is described by the formula (2):

$$(2) \qquad B = \prod_i x_i^{c_i}$$

Thereby, B denotes the parameter of the trait (for example the biomass), x the metabolite concentration and c the corresponding constants for all i metabolites, which are determined empirically by mathematical fitting of the correlation data. An example of a list of c values with respect to a canonical correlation analysis is depicted in Tab. 1.

It is to be understood that the metabolite data generated in the context of this invention may be normalized. The meaning of "normalisation" in this context is known in the art. An example how the generated metabolite data can be normalized is given in the appended examples.

Moreover, when a multiple regression method, particularly CCA, is to be employed, a missing value estimation is necessary. The meaning of "missing value estimation" in this context is also known in the art and a corresponding example is given in the appended examples.

As used herein, the term "evaluating" an MP based on the "correlation" determined by the corresponding methods of the present invention means that a given determined MP of a plant, for which the (potential for) expression of a desired trait is to be determined, is related to the results/outcome of these methods. The skilled person is readily in the position to put the step of "evaluating" into practice based on his common general knowledge and the teaching provided herein. Thereby, for example, the concentration data for the metabolites of (an) MP(s) are fitted into the model applied for the correlation analysis (e. g. model (2)) and the corresponding expression of the trait is calculated.

The result of the correlation analysis to be employed in the context of the present invention can be described as the highest possible correlation between combinations of metabolites (i. e. their amounts/levels) extracted from the MPs to be correlated and the (potential for) expression of the particular trait to be observed. For one particular embodiment of this invention, namely when the trait to be observed is biomass production/growth, the group of plant is a RIL population of *Arabidopsis thaliana* and the MPs comprise 181 certain metabolites, this highest possible correlation has a canonical correlation of 0.73 ($p < 10^{-64}$). Moreover, the results of the correlation analysis to be employed in the context of the present invention can be depicted as exemplarily shown in Figure 4 and the corresponding examples and tables.

For the evaluation step of an MP to be employed herein, any suitable analysis method can be used. The skilled person is readily in the position to find out such suitable analyses methods by his common general knowledge and the teaching provided herein. As a non-limiting example, such an analysis approach can be employed as it is exemplified in the appended examples.

In the context of the present invention, the term "deducing from the evaluation of an MP" means that from the outcome of the herein described step of evaluating an (potential for) expression of a trait is derived.

As mentioned above, based on his common general knowledge and the teaching provided herein, a skilled person is readily in the position to find out "correlation analyses" as well as "evaluating" and "deducing" approaches suitable to be employed in the context of the present invention. As mentioned, such analyses and approaches involve suitable statistical analyses of the data obtained in the context of the methods of the present invention. This refers to any mathematical analysis method that is suited to further process said data obtained. For example, these data represent the amount of the analyzed metabolites present in an MP of a biological sample, either in absolute terms (e.g. weight or moles per weight sample) or in relative terms (i.e. normalized to a certain reference quantity), the results of the analyses of the correlation between the MPs and the (potential for) expression of a trait as provided and described herein and/or the determined (potential for the) expression of a trait to be observed. In the context of this invention, normalization, e. g. to the total content of metabolites, or correction of background levels may also be employed. Mathematical methods and computer programs to be applied in context of the statistical analyses to be employed in the context of this invention can be find out by the skilled practitioner. Examples include SAS, SPSS and systatR. As mentioned, the statistically pre-treated data may, optionally, be subjected to a pairwise correlation analysis. Here series of pairs of data points from the analyzed compounds are looked at for correlation, whether positive or negative, for instance using Pearson's correlation coefficient.

In a preferred embodiment, the statistical analyses to be employed in the context of the methods of the invention furthermore involves network analyses. Network analyses, for example, aim at finding out higher order interplays of multiple factors on the basis of correlation data, e.g. pairwise correlation data. A comprehensive overview of methods for quantitatively analysing data obtained in context of the methods of the invention can be found in Fiehn (2001).

The term "group of plants" as used herein means any set of plants, wherein the plants comprised in said set share at least one common feature. As a non-limiting example, a "group of plants" in the context of the present invention may be a taxonomic unit or plants sharing at least one common feature of morphological, anatomical, physiological, ecophysiological, ecological and/or pathophysiological nature. The definitions as given herein above in the context of the definition of "trait" also apply, mutatis mutandis, to the at least one common feature, the plant of a "group of plants" share.

Accordingly, non-limiting examples for a "group of plants" that can be employed in the context of the invention are selected from the group consisting of a set of plants sharing the same kind of carbon fixation (like C4-, C3- or CAM-plants, and the like), a set of plants sharing the same kind of nitrogen fixation (like leguminoses, non-leguminoses, insectivore plants, and the like), a set of plants sharing the same kind of metamorphoses (like succulent plants, non-succulent plants, plants forming storage organs, and the like), a set of plants sharing the same anatomical structure (like plants with crown anatomy of the bundle sheeths, and the like) and plants producing the same kind of (economically desired) matter (like starch, protein, fat or sugar storing plants, woody plants, fibre plants, medical plants, and the like).

The skilled person is readily in the position to find out "groups of plants" that can be employed in the context of this invention, and furthermore, which particular plants or sub-groups of plants fall within these and the above exemplified groups of plants.

It is preferred in the context of the present invention that the term "group of plants" refers to plants which show high similarity among each other, particularly it is preferred that these plants show high genetic similarity among each other. It is of note that this is irrespective of the fact that it may be desired in the context of the present invention that a certain number of plants to be observed/screened show distinct differences in their genetic background which lead to differences in the (potential for) expression of a certain trait.

In view of the above, in a preferred embodiment of the present invention, the term "group of plants" refers to a taxonomic unit. The meaning of "taxonomic unit", particularly with respect to the plant kingdom, is known in the art. Non-limiting examples for plant "taxonomic units" are those corresponding to the taxonomic categories *phylum, subphylum, classis, familia, genus, species,* or even to a lower taxonomic category, such as a race, a variety, a cultivar, a strain, an isolate, a population, a *forma* or the like. Such corresponding "taxonomic units" for example are *Spermatophyta, Angiospermae, Dicotyledonae, Brassicaceae, Arabidopsis, Arabidopsis thaliana,* and the like. Generally, the taxonomic units of lower hierarchy are preferred in context of this invention. Accordingly, most preferably, the taxonomic unit corresponds to an isogenic line, for example to an RIL. In this context, "isogenic line" means, for example, a line with variance in only a limited number of genetic traits. Thereby, "genetic trait" refers to a character determined by a chromosomal region, a gene locus or, as it is preferred, to a gene or other nucleotide sequences.

It is of note that the above mentioned definitions of "taxonomic unit" are not limiting.

The systematic organisation, and hence further taxonomic categories and corresponding taxonomic units of the plant kingdom are known in the art. For example, this is described in Strasburger, Lehrbuch der Botanik (1991).

In general, the term "plant(s)" as used herein refers to an organism belonging to the plant kingdom, also known in the art as *plantae.* The systematic classification of the plant kingdom is known in the art (for example, see Strasburger, Lehrbuch der Botanik, 1991). Although it is envisaged herein that the term "plant(s)" generally refers to all organisms belonging to the plant kingdom, e.g. also to "plants" like cyanobacteria, algae or plant monads, it is preferred that (a) "plant(s)" to be employed herein belong(s) to any one of the taxonomic units *Bryophyta* (mosses), *Pteridophyta* (ferns) and *Spermatophyta* (seed plants). It is more preferred that a "plant"/"plants" to be employed herein belong(s) to the latter group, also known in the art as "higher plants". Within the "higher plants", the term "plant(s)" as used herein also refers to the therein comprised taxonomic units or sub-units (e. g. the taxonomic units or sub-units as defined above), for example to the taxonomic units or sub-units mentioned herein-above with respect to the definition of "group of plants". It is known in the art which further taxonomic units or sub-units are encompassed in the taxonomic unit "higher plants" and it is intended that these are also encompassed by the meaning of the term "plant(s)".

In one particular aspect of this invention, particularly with respect to the method for determining the correlation between the MPs and the (potential for) expression of a trait, the term "plant(s)" refers to any one of the taxonomic units *Spermatophyta, Angiospermae, Dicotyledonae, Brassicaceae, Arabidopsis* and *Arabidopsis thaliana,* and the like, or to taxonomic sub-units in between these taxonomic units. Also with respect to this particular aspect of the present invention, the taxonomic units of lower hierarchy are preferred.

In a further particular aspect of this invention, the term "plant(s)" refers to biomass producers, like, e.g., woody plants. Particularly but not limiting, said woody plants may be trees, preferably slow growing trees or trees producing hardwood.

Non-limiting examples for "trees" in the context of the present invention are trees of the genus *Quercus, Fagus, Acer, Fraxinus, Populus, Pinus, Salix, Eucalyptus, Aesculus, Platanus* or *Tilia.*

The meaning of "slow growing trees" is known in the art. For example, "slow" in this context refers to trees that are to be grown for at least 5, preferably at least 10, more preferably at least 20, more preferably at least 50 and most preferably at least 100 years until their harvest. For example such slow growing trees belong to the genus selected from the group consisting of *Quercus, Fagus, Acer* and *Fraxinus.* Whether a particular tree is a slow growing tree is known in the art. The meaning of "trees producing hardwood" is also known in the art. For example, "hardwood" in the context of the present invention means wood having a basic density of at least 550 kg/m$^3$, more preferably at least 560 kg/m$^3$, more preferably at least 600 kg/m$^3$ and more preferably at least 640 kg/m3. Whether a particular tree is a tree producing hardwood is also known in the art.

It is further known in the art that, usually, "slow growing trees" are trees that produce hardwood. For example hardwood producing trees may be trees of the above mentioned genera. Other "slow growing trees" or "trees producing hardwood" may be mahogany trees, birches, elm trees, locusts or hornbeams, and the like.

As mentioned above, the methods of the present invention are particularly useful when slow growing plants are to be monitored. For example, this means that such plants have to be grown for a relatively long period of time until they are harvested. Additionally, the methods provided and disclosed herein are particularly useful where plants, particularly trees, are to be monitored, the adult biomass or biomass production/growth rate of which cannot readily be estimated in early developmental stages. Without being bound by theory, examples of such plants are the above mentioned "slow growing" or "hardwood producing" trees. It is also envisaged herein, that such plants, particularly trees are encompassed by the meaning of "plant(s)" or "group of plants".

The culturing of such above-mentioned plants may be particularly time and cost intensive. Therefore, particularly when plants like the above-mentioned plants are to be monitored, the methods of the present invention are extraordinarily useful.

However, in this context it is to be understood that the herein provided methods can also be applied to other plants, e.g. to comparably fast growing plants, like, e.g. crops or fast growing trees, without any methodological limitations.

In a preferred embodiment, the plant to be tested for its (potential for) expression of a desired trait belongs to the same group of plants (e.g. the same species) which was employed in the corresponding method for determining the correlation between the MPs and the (potential for) expression of said trait. However, it is also envisaged herein that the provided methods for determining or predicting the expression of a trait provided herein can also be generalized to plants belonging to other groups of plants. In other words, the plant(s) the expression of a trait of which is to be determined or predicted by the corresponding methods of the invention, may also belong to another group of plants (e.g. another species) than the group of plants which was employed in the corresponding method for determining the correlation between the MPs and the (potential for) expression of said trait.

For example, the correlation analysis may be firstly performed in a plant belonging to a certain group of plants which allows, e. g., to carry out the correlation analysis in an economically efficient, statistically significant and highly reliable and/or standardized manner (for example, and as also exemplified herein, by using plants of the species *Arabidopsis thaliana,* e. g. by using RILs of *Arabidopsis thaliana*). In contrast thereto, the plant(s), in which the expression of the desired trait is to be determined or predicted, may be of another group of plants, like for example biomass producers (or other species of high economical relevance).

However, it is preferred herein that the plant(s) in which the expression of the desired trait is to be tested, is/are of the same group than the plants for which the correlation analysis be performed or is/are at least of a closely related group of plants (e.g. the same family, preferably the same genus, more preferably the same species).

It is envisaged herein, that "(a) plant(s)" as employed and monitored herein is (are) one individual plant, a pool of more than one plant, (a) part(s) or (an) organ(s) of (a) plant(s), a seed, a seedling, an adult plant (e.g. in its vegetative, flowering or fruiting state), tissue of a plant, a cell or a pool of cells of a plant (e.g. a cell culture of a plant), and the like. As mentioned, in one particular aspect of the present invention, the "plant(s)" to be monitored, particularly those "plant(s)" the MP(s) of which is determined in the context of the methods provided herein, is a part of (a) plant(s). Preferably this "part" is such that its separation from a living plant does not, or only to a low extent, negatively influence the further development of said plant. Non-limiting examples of a "part" of a plant as employed in the context of this invention are leave, stem, flower, fruit, bud, branch, and the like. As already mentioned herein, when taking advantage of such a "part" of a plant, the corresponding methods provided herein can be low invasive methods.

It is to be understood that the definitions of the term "plant(s)" given herein-above, likewise apply for plants of a "group of plants" as defined herein, as well as for (a) plant(s), for which the (potential for) expression of a trait or (an) MP(s) is to be determined in context of this invention.

The number of the "plants" of which the (potential for) expression of a trait is determined or the MPs of which are determined in the context of the herein provided methods for determining a correlation, preferably is intended to be high. High amounts of plants to be monitored in the context of the mentioned methods lead to more statistically significant and reliable outcomes, i.e. results, of said methods for correlation, and therefore render said methods more powerful with respect to their explanatory power.

"High" in this context means, for example, at least 3, at least 5, at least 7, at least 10, at least 20, at least 50, at least 100, at least 300, at least 400, at least 700 or at least 1000 plants, wherein the higher numbers are preferred.

It is of note that the steps of "determining the (potential for) expression of a trait" and "determining the MPs of plants" being comprised in the herein provided methods for determining the correlation between the MPs and the (potential for) expression of a trait may be applied at the same point in time, but also at different point in times. Particularly, it is intended that the step of "determining the (potential for) expression of a trait" is applied at a later point in time as the step of "determining the MPs". Such an approach would result in the correlation of a current metabolic state), i. e. (a) current MP(s), of (a) plant(s) with a future expression of a trait.

For example, the point in time when the step of "determining the MPs of plants" is to be applied may be that point in time, when, according to the well established practice of plant breeding approaches, plants, particularly young plants, are to be selected for further breeding.

For example, the point in time when the step of "determining the (potential for) expression of a trait" is to be applied may be that point in time, when a certain expression of a trait is desired to occur, which, for example, may be the time of harvest of the plant.

However, it is preferred herein that the steps of "determining the (potential for) expression of a trait" and "determining the MPs of plants" are applied at the same point in time, for example, at that point in time when plants are to be selected for further breeding.

In a further aspect the present invention refers to a method for determining or predicting the biomass production/ growth rate of a plant comprising the steps of:

(a) determining the MP of said plant;
(b) evaluating said MP based on the results of the correlation analysis between MPs and (the potential for) biomass production/growth rate of a group of plants as described herein and as demonstrated in the appended examples; and
(c) deducing from said evaluation of (b) the biomass production/growth rate exhibited by said plant.

As also mentioned above, such results are exemplarily depicted in Figure 4 and the corresponding examples and tables (e. g. in form of the highest possible correlation between combinations of metabolite levels extracted from said MPs and said (potential for) biomass production/growth rate).

As demonstrated in the appended examples certain metabolites comprised by the MP(s) determined herein, show particularly high correlation with the expressed biomass production/growth, e. g. they are highly ranked in the canonical correlation analysis exemplified herein, which means that they have high absolute numerical COR values. As also demonstrated in the appended examples, these so-called "main drivers of correlation" may, as non-limiting examples, belong to one or more of the following groups of metabolites: Metabolites of the central carbon or nitrogen metabolism, metabolites of membrane and/or (phospho)lipid biosynthesis, metabolites of nitrogen assimilation, metabolites of the stress response, metabolites acting as plant hormones, metabolites acting as (second) messengers and/or metabolites of the secondary metabolism of plants.

Based on the teaching provided herein (see, for example, the appended examples), i. e. that and how these "main drivers" correlate with biomass production/growth, it is possible to determine or predict the expression of biomass production/growth of any plant which is desired to be tested by taking advantage of the means and methods provided by the present invention and the correlation analyses and the results of the same, respectively, provided herein.

In the context of this further aspect of the invention, the meaning of the terms "evaluating" and "deducing" as described herein-above applies, mutatis mutandis.

Moreover, in the context of this method, it is again preferred that the plant desired to be tested belongs to the same, or at least to a closely related group of plants, as the plants for which the correlation analysis were performed and exemplified herein (*Arabidopsis thaliana*). For example, it is preferred that the plant to be tested is also a C3-plant, more preferably also a plant not belonging to the group of Leguminoses, more preferably a dicotyledonous plant and/or even more preferably a plant belonging to the group of *Brassicaceae.*

In a further embodiment, the present invention relates to a method for breeding of a plant comprising the steps of:

(a) determining or predicting the expression of a trait of said plant according to the corresponding methods provided herein; and
(b) selecting said plant on the basis of the expression of said trait determined or predicted by (a).

The method for breeding of a plant as provided and disclosed herein takes advantage of, i.e. comprises, the methods for determining or predicting the expression of a trait of a plant as disclosed herein. Accordingly, the definitions given herein with respect to these methods for determining or predicting apply here, mutatis mutandis.

It is to be understood that in the methods for breeding of the present invention, plant breeding approaches as known in the art, or (a) step(s) thereof, may, at least partially, be comprised in addition to or in combination with the methods for determining or predicting the expression of a trait of a plant of the present invention. It is preferably envisaged herein that such methods of the art, or (a) step(s) thereof may, at least partially, be replaced by the methods for breeding of the present invention.

Various plant breeding approaches are known in the art.

For example, plant breeding approaches of the art or the corresponding methods for breeding usually comprise some kind of selection step for the plant to be bred (and/or for progenitor lines and/or offspring thereof), and hence to be further treated (like, for example, to be further cultured). The criterion, on the basis of which a plant is selected, or at least one other plant is excluded, usually is a desired (potential for) expression of (a) desired trait(s) (e. g. such as yield/biomass production or robustness against biotic or abiotic stress, and the like). This mentioned (potential for) expression is thereby usually analysed/determined by suitable means and methods known in the art. Non-limited examples of such means and methods are counting seeds, grains, leaves, flowers, bids, fruits, and the like, or weighting plants or parts thereof, like seeds, grains, leaves, flowers, buds, fruits, and the like.

A further non-limiting example of a method for analysing/determining a(n) (potential for) expression of a trait is the application of some kind of selective pressure on (a) plant(s) to be selected or bred and subsequently selecting the (a) plant(s) on the basis of its (their) respond to said selective pressure. Such kind of methods are known in the art. For example, such method may comprise a step of contacting (a) plant(s) to be selected/bred with an antibiotic agent (e.g. an agent selective for a certain marker or resistance present in said plant(s)) and subsequently selecting this (those) plant(s) that are resistant against said antibiotic agent (e.g. due to the presence of a corresponding marker for resistance in the plant(s)). In this context, non-limiting examples of "selective pressure" are the application of a stress, like, e.g. drought, hypoxia, light stress, heat stress, cold stress, nitrogen deficiency, presence of pathogens (e.g. nematodes, viruses, bacteria, fungi, and the like), limited soil availability, deficiency or excess of micro- or macro nutrients, application of toxic substances (e.g. heavy metals, and the like), and the like. Non-limiting examples of the plant's/plants' "response" to such applied selective pressure are decease, reduced or degenerated growth, discolouring of leaves, flowers, fruits, reduced or degenerated production of leaves, flowers, fruits, and the like.

Moreover, plant breeding approaches of the art can comprise steps of crossing and/or selfing progenitor lines. The meaning of "crossing" and "selfing" with respect to this matter is known in the art. The skilled person is aware how steps of crossing, selfing and selecting are to be carried out to meet the specific requirements of the different plant species.

As mentioned above, the breeding method of the invention may include all sorts of breeding techniques, or (a) step(s) thereof, the skilled person commonly applies in order to achieve plants with a certain desirable (potential for) expression of desired traits, e.g. agronomical traits, such as new plant varieties or cultivars, elite lines and, in particular, commercial plant varieties. In one particular aspect, the method for breeding of a plant as provided herein is intended to be a plant breeding approach, comprising a step of selection on the basis of the (potential for) expression of a desired trait determined (or predicted) by the corresponding methods provided herein. Accordingly, it is preferred in context of the herein provided breeding methods that a step of selecting as known in the art, for example a step of selecting as described above, is replaced by the step of selecting as defined herein.

The breeding methods of the invention may also comprise steps including the application of techniques that are generally considered as being unconventional, such as interspecific crossing or the propagation of a progenitor or pedigree generation by way of non-sexual processes including, for instance, in vitro propagation using cell culture methods as known in the art. It is furthermore envisaged that the breeding methods of the invention may also encompass the use of one or more transgenic lines for instance as progenitor lines. Said one or more transgenic lines may be transformed with further traits, for example in addition to the trait, the (potential for) expression of which is desired to be investigated. However, it is preferred that the present breeding methods are carried out according to conventional breeding methods, i.e. without the use of genetic engineering. This meets the demand of the consumers for non-genetically engineered crops. Moreover, the present breeding method provided herein may also include the production of hybrid seed as is for example customary practice with crops like maize.

In the context of the methods for breeding of a plant as disclosed herein, the term "selecting a plant on the basis of the expression of a trait" refers to a step of a plant breeding approach, where the expression of a desired trait was determined or predicted by the corresponding methods of the present invention and said plant is then selected by considering whether its so determined/predicted expression of said trait meets a desired standard (e. g. a desired size, weight, length, amount, number, and the like).

According to the present invention, the step of "selecting" may be carried out at each suitable stage of the breeding process. For instance, it may be used for screening for progenitor lines with which the breeding is started. Alternatively, it may also be used for selecting those plants of a segregating progeny which desirably expressing (the potential for) a trait to be observed. For example, the latter case is particularly relevant when germplasm is introduced into the breeding process and it is known that at a preceding stage the plant to be bred do not contain the genetic background for a desired trait, as is for example the case with many North American maize lines.

In a further embodiment, the present invention relates to a method for identifying one or more quantitative trait loci/locus (QTL(s)) for a trait or for the potential for expression of a trait of a group of plants comprising the step of identifying (a) QTL(s) for metabolite combinations (herein also referred to as "multiple metabolite QTL(s)"), particularly for metabolite combinations comprised in an MP as defined herein. Thereby, it is intended that the metabolite combinations (or the corresponding MP) show(s) strong correlation with the (potential for) expression of said trait of said group of plants. It is preferred that said correlation is determined by the methods of determining the correlation between the MPs and the (potential for) expression of a trait as provided herein.

Particularly, when the trait, for which or for the potential for the expression of which QTLs are to be identified, is biomass production/growth rate, it is preferred that said metabolite combinations are deduced from the herein provided results of a corresponding correlation analysis. As already mentioned herein, an example of such results is depicted in Figure 4 and the corresponding examples and tables. From these particular results, and also from the results of the method for determining the correlation between the MP and the (potential for) expression of a trait as provided herein, the skilled person is readily in the position to deduce all information necessary to figure out the metabolite combination(s) for which (a) QTL(s) is to be identified in order to indirectly obtain (a) QTL(s) for (the potential for expression of) a desired trait, like biomass production/growth rate.

The meaning of the term "quantitative trait locus/loci (QTL(s))" is known in the art. The term refers to a locus on a chromosome that is associated with a certain trait.

Also known in the art are standard methods how (a) QTL(s) for (a) desired trait(s) can be identified.

Usually, methods for the identification of a QTL(s) take advantage of a set of data corresponding to a desired trait (for example data for metabolites comprised in a MP), genetic markers and a, preferably already known, linkage map thereof. These tools are applied in chromosome mapping approaches, eventually leading to the identification of (a) QTL(s) corresponding to the desired trait.

QTL identification approaches can further be supported by suitable computer applications and corresponding software (e. g. software packages like PLABQTL (Utz, J QTL 2 (1996)) and QTL-Cartographer (Basten, Zmap - a QTL cartographer.

eds. Smith, C. et al. Proceedings of the 5th World Congress on Genetics Applied to Livestock Production: Computing Strategies and Software 22, 65-66. 1994. Guelph, Ontario, Canada, Organizing Committee, 5th World Congress on Genetics Applied to Livestock Production.)).

A non-limiting example of a method for identifying QTL(s) as envisaged and provided in the context of the present invention is given in the appended examples. For instance, in Example 5, a meta QTL search was performed using the biomass vector (canonical variate) as a (new) trait in order to indirectly determine (a) QTL(s) for biomass production/growth rate. Accordingly, the term "metabolite combination(s) showing strong correlation with the (potential for) expression of a trait" also refers to a vector of a desired trait (e. g. the canonical variate), for example a biomass vector as deduced from a method for determining the correlation between MPs and the (potential for) expression of a trait as disclosed herein.

The skilled person is readily in the position to adapt the herein exemplified and above-described indirect identification of (a) QTL(s) for (the potential for expression of) biomass production/growth rate to any other desired traits, by the teaching provided herein and his common general knowledge.

In the context of this embodiment, the term "metabolite combination(s)" means (a) set(s) of at least 2, at least 5, at least 10, at least 20, at least 50, at least 100, at least 200, at least 500 or at least 1000 metabolites, wherein the higher numbers of metabolites are preferred. In this context it is of note that the herein given definitions for "metabolite (s)", for example the definitions of "metabolite(s)" given with respect to "MP(s)", apply here, mutatis mutandis. For example "metabolite combinations" to be employed in the context of the present invention can be deduced from the appended examples, particularly from Table 1. A particular non-limiting example of such a "metabolite combination" are the first 3, 5, 7, 10, 12, 15, 20, 30 or 40 metabolites, preferably known metabolites, of Table 1. Thereby, the higher numbers are again preferred.

Further, in the context of this embodiment, the term "strong correlation" means that the metabolites to be comprised in the metabolite combination(s) to be employed are highly ranked (either negatively or positively) in a correlation analysis between MPs and the (potential for) expression of a trait as provided herein. In this context, "highly ranked" means that a metabolite comprised in said MPs has a high absolute value of correlation ("COR"), preferably by a low corresponding p-value ("PV"). The corresponding definitions of the terms "high" and "low" are given herein in the context of the definition of the term "correlation" and apply here, mutatis mutandis.

The QTLs for a certain trait usually are the same as the QTLs for the potential for expression of said trait. However, it is also conceivable, and therefore also envisaged herein, that there are certain QTLs existing which do not contribute to the status quo of the expression of a trait at a certain point in time, but which represent the genetic background for a future expression of said trait, which means that they contribute to the potential for expression of said trait. For example, a first QTL that mainly contributes to dry weight production of a tree's seedling, may not, or to a lower extent, contribute to the dry weight production of the same tree in the adult state. In this state, a QTL that mainly contributes to dry weight production may be a different, second QTL. For example said first QTL may be a QTL contributing to cellulose production, whereas said second QTL may be a QTL contributing to lignin production.

In this context, it is also of note that, in principle, the same applies for the MPs to be employed herein representing a certain (potential for) expression of a trait.

Definitions of the terms "trait", "plant(s)", "group of plants", "metabolites", "(potential for) expression", "correlation" are already given herein and also apply with respect to the above described further embodiment of the present invention, mutatis mutandis.

Moreover, in a further embodiment, the present invention relates to a method for identifying a candidate gene involved in the determination of the expression or the potential for expression of a trait of a plant. Said method comprises the step of isolating or identifying a gene corresponding to any one of the QTLs as identified by the corresponding methods described and provided herein.

This embodiment makes use of the contribution of the present invention for approaches aiming either at the identification of novel genes or at the identification of novel, i.e. not known, functions of already known genes. Accordingly, the term "identifying" a candidate gene refers to both of these aspects.

It is preferred that the genes to be "identified" are of particular industrial/commercial interest, i.e. that they are involved in the determination of a trait, the (potential for) expression of which is of great economical, for example agroeconomical, interest.

In the context of the present invention, the term "candidate gene", inter alia, refers to a gene that is involved in the determination of a trait, and, therefore determines the (potential for) expression of a trait.

Particularly, a "candidate gene" as employed herein is a gene that corresponds to a certain QTL, preferably a QTL for a trait of high (agro)economical interest. With respect to this, "corresponding" or "correspond(s)" means that a QTL, which is known in the art to be a locus on the chromosome contributing to, i.e. determining, a trait, comprises the gene. Accordingly, once a QTL for a trait of interest is known, for example when it was identified according to the corresponding methods of the invention, the corresponding gene can be identified by the corresponding method provided herein. It is envisaged that this method of the invention comprises a step of isolating or identifying a gene corresponding to an identified QTL. For example, said step takes advantage of corresponding "isolation/identification" methods known in the art. Examples of such " isolation/identification" methods are chromosome mapping methods, and the like. Examples of these and also of further of such "isolation" methods are known in the art.

With respect to this embodiment, the term "involved" reflects that one or more than one gene determines a trait and thereby its (potential for) expression.

The definitions concerning the terms "determination", "expression", "trait", "plant" etc. as given herein-above, also apply to the corresponding terms as used in the context of this particular embodiment.

In one aspect of the above-described embodiment, the candidate gene can be identified by taking advantage of the knowledge of the metabolite combination(s), for which the corresponding QTL was identified. From the knowledge of one or more metabolite(s) being comprised in said metabolite combination(s), in combination with the knowledge of the biochemical pathway said one or more metabolite(s) is (are) involved in, (a) enzyme(s) of said biochemical pathway corresponding to said one or more metabolite(s) can be identified. In this context, "corresponding to said one or more metabolite(s)" means that said metabolite(s) are involved in the reaction catalyzed by said enzyme, for example as substrate or reaction product of the enzyme. In a subsequent step, from said enzyme(s) the corresponding encoding candidate gene can then be deduced.

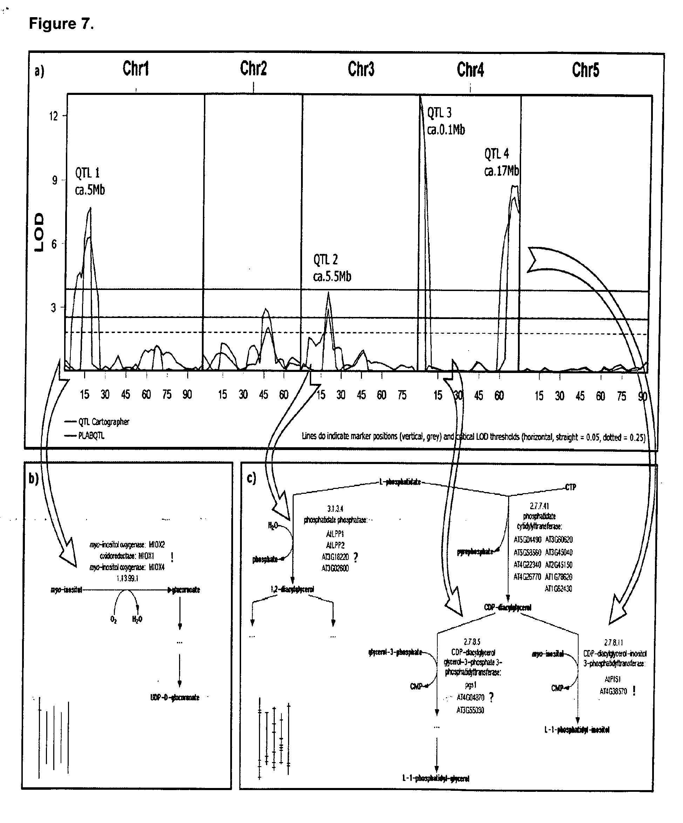

In such a method for identifying a candidate gene, the often time consuming step of isolating or identifying a gene corresponding to an identified QTL can so be simplified. Particularly, such a method, at least partially, obviates time consuming "isolation" methods like, or example, chromosome mapping methods. A non-limiting example of such a method for identifying a candidate gene is given in the appended examples, e.g. in Example 4 and Figure 7. In this example, an analysis of detected metabolic QTLs with respect to underlying biochemical pathways demonstrates that it is possible to identify candidate genes even at a rather low mapping resolution. Irrespective of the fact that the mapping resolution is still limited, it was demonstrated herein that an analysis of the metabolic QTL with respect to candidate genes as derived from known biochemical pathways is surprisingly fruitful. For example, it was exemplified herein, that an inspection of the myo-inositol pathway allows the identification of candidate genes within the region of all myo-inositol QTL identified (see appended examples).

The present invention also refers to a method of screening for a plant that exhibits a desired expression or potential for expression of a trait comprising the step of determining or predicting the expression of said trait according to a corresponding method provided and exemplified herein. This screening method of the present invention may for example be applied in agricultural plant breeding approaches.

Generally, this embodiment relates to approaches, which require to identify in a pool of plants those plants which have a desired feature, i. e. a desired (potential for) expression of a trait. To evaluate, whether a plant has the desired feature, the herein provided methods for determining or predicting the expression of a trait are to be employed. Thereby, it is envisaged, that, these methods for determining or predicting may either, at least partially, obviate corresponding methods for determining or predicting the expression of a trait as they are known in the art, or may supplement or support those already known methods. Particularly, when these known methods are time and cost consuming and/or result in weakly reliable outcomes, it is preferred that they are replaced by the herein-provided and above-described methods for determining or predicting.

However, in contrast, to the screening methods of the art, those of the present invention take advantage of the methods for determining or predicting the expression of a trait as provided and described herein.

In one particular aspect, the provided method of screening for a plant may further comprise prior to the step of determining a step of subjecting plants, and accordingly the plant to be screened for, to a certain treatment. For example, this treatment is intended to influence a feature of a plant, i. e. the (potential for) expression of a trait, in a desired manner or to create (a) new (desired) feature(s).

The definition of the term "treatment" as given herein-below, applies here, mutatis mutandis.

Furthermore, the present invention refers to a method of determining whether a treatment influences the expression or potential for expression a trait of a plant. Said method comprises the steps of:

(a) subjecting (a) plant(s) to a treatment;
(b) determining or predicting the expression of said trait according to a corresponding method provided and described herein; and
(c) comparing said determined or predicted expression of said trait with the determined or predicted expression of said trait of (a) control plant(s) which has not been subjected to said treatment.

In the context of this embodiment, the term "treatment" refers to any kind of influence of the environment/ environmental factors on a plant, particularly on the (potential for) expression of (a) trait(s) of said plants. Hereby, the term "influence of the environment/environmental factors" is intended to also encompass "man made" influences, which, for example, where applied to the plant consciously or unconsciously. For example, such an influence can be the application of a toxin from polluted air, water or soil, and the like.

In one aspect of the present invention, the above-described methods of determining the influence of a treatment may particularly be applied in approaches that require to know or to find out if or how (an) environmental factor(s) influence the (potential for) expression of a trait. For example, this may be of relevance in particular applications of the herein provided methods, where it is desired to find out differences between several plants in the genetic background that determines a trait, and where, accordingly, it is required that the influence of the environment/environmental factors is kept equal between said plants and/or during the observation period. Accordingly, in such applications, the above-described methods of this particular embodiment of the present invention can be used to validate whether a certain (potential for) expression of a trait of a plant is indeed due to a certain (difference in the) genetic background of said plant.

In a further aspect of the above embodiment, it is envisaged that the provided method of determining the influence of a treatment is applied in plant breeding approaches, particularly in such approaches, where it is desired that the (potential for) expression of a trait is manipulated. One particular example of such an approach comprises the treatment of (a) plant(s) with radiation or chemical mutagens. Particularly such treatments may result in an alteration of the genetic background that determines a trait, for example by introducing (a) mutation(s) into genes that determine a certain trait. In such a case, the methods of the above specific embodiment are particularly useful to identify (a) plant (s) in which a certain (potential for) expression of a trait is due to changes in the genetic background (independent from the influence of environmental factors).

Particular examples of a "treatment" to be employed in the context of this embodiment may be selected from the group consisting of light/dark treatments; drought/moisture treatments (in the substrate and/or in the air); heat/cold treatments; substrate composition treatments; treatments with varying space for rooting; treatments with varying macronutrients and/or micronutrients, radiation treatments, treatments with chemicals (like, e.g., mutagens) and treatments with pathogens.

In one particular aspect of this invention, it is intended that the herein provided methods of screening for a plant and/or methods of determining the influence of a treatment are comprised in or are combined with the methods of breeding of a plant.

These and other embodiments are disclosed and encompassed by the description and examples of the present invention. All of the publications, patents and patent applications referred to in the specification in order to illustrate the invention are hereby incorporated by reference in their entirety. Further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.google.de. An overview of patent information in biotechnology and a survey of relevant sources of patent information useful for retrospective searching and for current awareness is given in Berks, TIBTECH 12 (1994), 352-364.

Furthermore, the term "and/or" when occurring herein includes the meaning of "and", "or" and "all or any other combination of the elements connected by said term".

The present invention is further described by reference to the following non-limiting figures and examples.

The Figures show:

**Figure 1**    **Distribution of shoot dry mass in the recombinant inbred line (RIL) population**
Shown is the mean biomass (mg/plant) estimated by REML. The arrow indicates the biomass determined for the parental lines C24 (1.265 mg/plant) and Col-0 (1.254 mg/plant). The histogram of the shoot dry mass of the RIL testcrosses to the parents is shown in the inset.

**Figure 2**    **Distribution of metabolic and biomass QTL**
Significant metabolic QTL of metabolites known by structure are shown as black boxes at marker positions if covered by support interval. For simplicity, QTL of metabolites of unknown structure are omitted here. Information on all detected QTL is given in Table 2. Metabolites are gray-scale-coded according to their chemical group as denoted on right side. Vertical lines indicate marker positions - several of which are labeled with approximate distance in cM (top). Asterisks indicate QTL 'hot spots' (as determined through 1000 permutations on a 0.05 level).

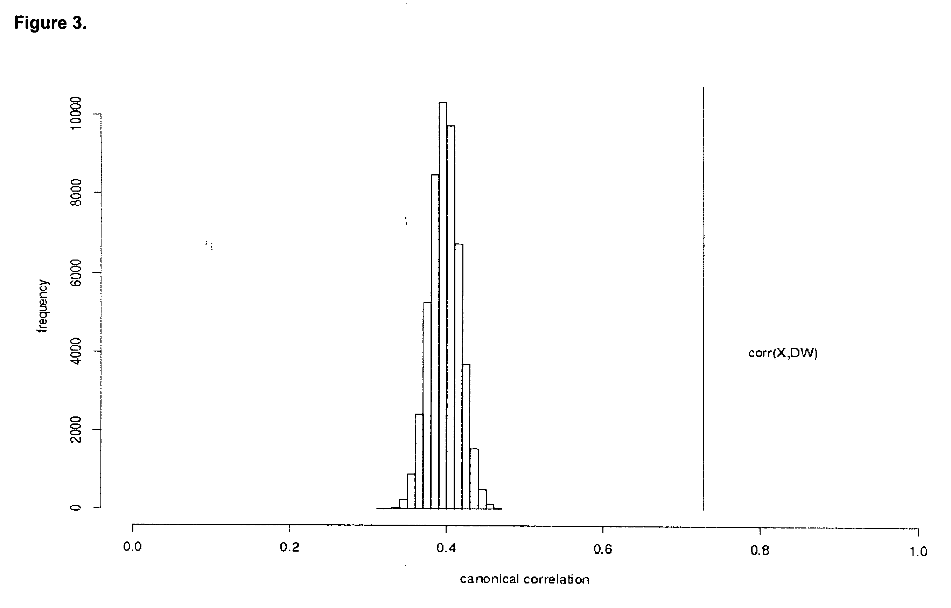

**Figure 3**    **Histogram of canonical correlations between the metabolite matrix and random permutations of the dry weight vector**
The line on the right corresponds to the canonical correlation between the actual dry weight vector (DW) and the actual metabolite matrix (X)R.
The distance to the median of the random correlation amounts to 17 standard deviations.

**Figure 4** **Relation between actual dry weight and dry weight predicted by the metabolite matrix in cross-validation**
Size of the training set was 1020, the 124 data points of the test set are displayed. The diagonal represents the exact prediction.

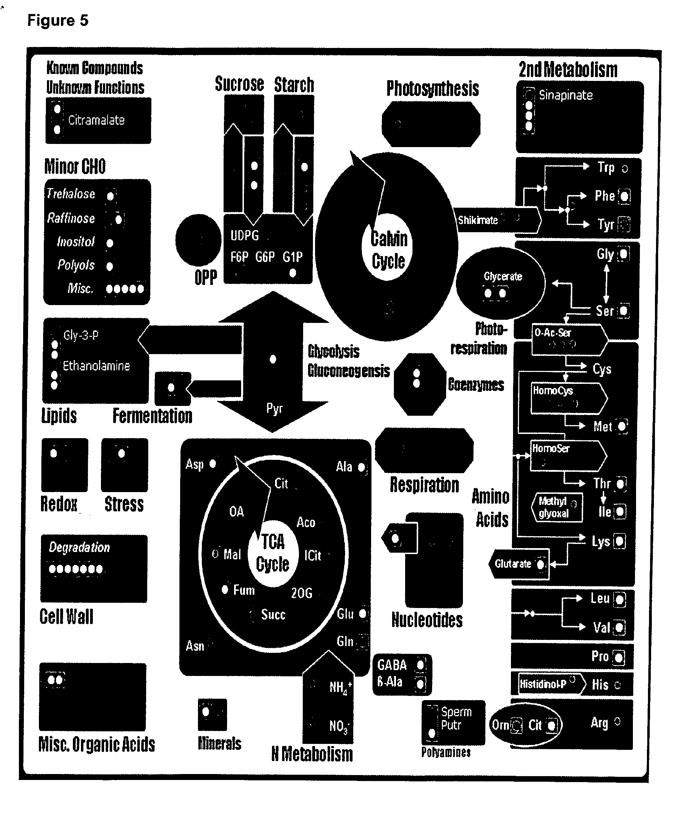

**Figure 5** **Representation of the most important metabolites known by structure according to CCA on biochemical pathways**
This representation of metabolism indicates all known metabolites we analyzed using GC/MS that could be annotated in MapMan (Thimm, Nat Biotechnol 18, 1157-1161 (2000)). Red color visualizes metabolites which are high ranked in CCA (positions 1-44).

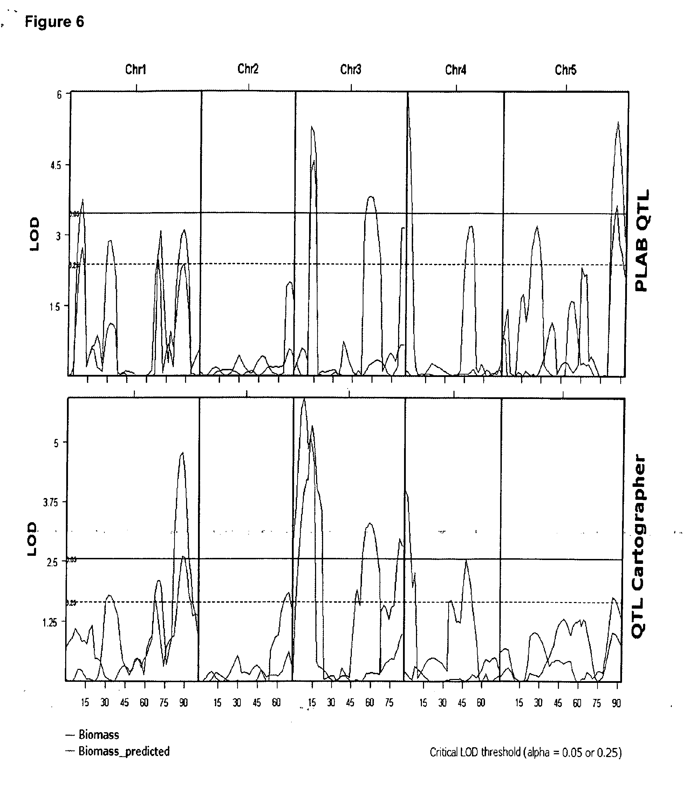

**Figure 6** **LOD profiles of actual (blue) and predicted (red) dry weight estimated by PLABQTL (top) and QTL-Cartographer (bottom)**
Critical LOD thresholds for 0.05 and 0.25 are indicated by full and dotted lines, respectively.

**Figure 7** **The myo-inositol LOD profile indicates 4 significant QTL (a)**
Analysis of present knowledge about biochemical pathways containing myo-inositol (according to TAIR) reveals candidate genes for all four QTL. Excerpts are shown for inositol oxidation (b) and phospholipid biosynthesis (c). Arrows point from the respective QTL region to the reaction step of the co-localized enzyme. Two of these marked reaction steps directly involve myo-inositol ("!": myo-inositol oxygenase, CDP-diacylglycerol-inositol-3-phosphatidyltransferase) while two others represent enzymes from the same pathway. ("?": phosphatidate phosphatase, CDP-diacylglycerol glycerol-3-phosphat 3-phosphatidyltransferase).

The Examples illustrate the invention.

**Example 1: Material and methods**

Creation of recombinant inbred line (RIL) population:

Two reciprocal sets of RILs have been developed from a cross between the two *A. thaliana* accessions C24 and Col-0. $F_2$ plants were propagated by controlled self-pollination using the single-seed descent method to the $F_8$ generation, where genotyping and bulk amplification was performed. The mapping population consisted of 228 Col-0xC24 $F_8$ and 201 C24xCol-0 $F_8$ individual lines. The RIL population was genotyped with a set of 109 framework SNP markers (Törjé, Plant J 36, 122-40 (2003)) as described elsewhere (Törjék, Theoretical & Applied Genetics (2006)). Marker distributions per chromosome in the two subpopulations were compared with Mantel tests (1000 permutations) of the corresponding similarity matrices obtained by simple matching, using the statistical software package Genstat version 6.1.

Plant Cultivation:

For the RIL population an incomplete block design with 54 blocks and 4 replicates was used. Plants were grown in 1:1 mixture of GS 90 soil and vermiculite (Gebrüder Patzer, Sinntal-Jossa, Germany) in 96-well-trays. Six plants of the same line were grown per well. Seeds were germinated in a growth chamber at 6°C for two days before transfer to a long-day regime (16 hours fluorescent light [120 $\mu$mol m$^{-2}$ s$^{-1}$] at 20°C and 60% RH / 8 hours dark at 18°C and 75% RH). To avoid position effects, trays were rotated around the growth chamber every two days.

Shoot dry biomass:

Shoot dry biomass was determined 15 days after sowing (DAS). Plants from the same well were harvested together and placed in a vacuum oven at 80°C for 48 hours. Dry biomass was measured using an analysis balance. Mean shoot dry biomass in mg/plant per plant was estimated using the linear mixed model ((Piepho, J Agron Crop Sci 189, 310-322 (2003)) G + E : E*G + E*GC + E*GC*T, where E is experiment, G is genotype, GC is growth chamber, T is tray (REML procedure in Genstat). Biomass in the two subpopulations was compared with a two-sided t-test.

Metabolite data:

Sample preparation, measurement and data processing:

Samples for the analysis of metabolic composition were collected together with the material for dry biomass analysis at 15 DAS. Harvested material (shoot and leaf) was cooled below -80°C immediately and kept at this temperature until further processing. Derivatization, GC/MS analysis and data processing were done as described elsewhere (Lisec, Nature Protocols, In Press (2006)).

The resulting data consist of unique mass intensity values for each referenced compound and measurement respectively. These raw data were normalized and otherwise directly used for QTL analysis.

Normalization:

Metabolite data were normalized by dividing each raw value by the median of all measurements of a day for one metabolite.

Missing value estimation:

For the CCA missing value replacement is necessary. The 6% missing values in the metabolite matrix were imputed with a SOM algorithm Vesanto, (SOM Toolbox, A57 Toolbox for Matlab 5i Technical Report (2000)). The mean square error was estimated by the comparison of known values with those calculated from the SOM algorithm. The coefficient of variation (root mean square error divided by the mean) was 0.3.

QTL analyses:

Two software packages implementing different detection algorithms (PLABQTL: multiple regression (Utz *J YTL* 2 (1996)); QTL Cartographer: maximum likelihood methods (Basten, Computing Strategies and Software 22, 645-66 (1994)) were combined to obtain robust QTL estimates. Composite interval mapping (CIM) was performed on a RIL population of 429 lines (dry biomass) or 369 lines (metabolites) with 1 cM increments. Cofactors were automatically selected by forward stepwise regression. Significant LOD thresholds were determined by 5000 permutations. QTL were regarded as significant, if they were detected with $LOD_{0.05}$ in one package, and reached at least $LOD_{0.25}$ in the other. QTL location and partial $R^2$ were further validated using 1000 runs of the 5-fold cross-validation procedure implemented in PLABQTL. Given a population size of 429 and a significance level of 0.05, it was show (Hackett, Plant Mol Biol 48, 585-599 (2002)) that 99% of all QTL which contribute more than 5% to the total variance and more than 50% of those which contribute more than 1% will be detected. Most of the undetected QTL will be below the 1% line. To obtain the same number of QTL which have a contribution of 0.5% the population would have been doubled.

Co-localizations of QTL from different traits should be expected given the high number of traits and the limited number of markers. The deviation from the random number of co-localizations was calculated as follows: The QTL of each metabolite were randomly distributed over the 105 marker positions. Then the number of co-localizations with each of the dry biomass QTL or with other metabolite QTL was counted. This procedure was repeated 1000 times, yielding a distribution of the maximum numbers of co-localizations. The 95% quantile of the distribution for metabolite-biomass QTL co-localization was eight, hence eight or more QTL at one genome position are regarded as significantly co-localized. The corresponding 95% quantile for the metabolite-metabolite QTL co-localization was thirteen.

Integrated analysis of phenotypic and metabolite data:

Linear models for the relation between metabolite profile and biomass:

The relation between biomass and metabolite profile was measured by simple Pearson correlation between the dry biomass and all metabolite concentrations and by a more complex multiplicative model. The first corresponds to the following model, referred to as model 1:

$$(1) \qquad B = c_i x_i .$$

The second model can be described by:

$$(2) \qquad B = \prod_i x_i^{c_i}$$

*B* denotes the biomass, *x* the metabolite concentration and *c* the corresponding constants for all i metabolites.

Multivariate linear analysis:

The canonical correlation analysis calculates the highest possible correlation between linear combinations of the columns from two matrices with the same number of rows. If the second matrix has only one column, this corresponds to a ordinary least square (OLS) regression. The correlation thus found is called canonical correlation, the corresponding linear combination canonical variate. The mathematical foundation is described in the literature Hotelling, J Educ Phsychol 26, 139-143 (1935)), (Kuss, Max Planck Institue for Biological Cybernetics, Technical Report 108, (2003). The R function *cancor* was used to calculate the canonical correlation between metabolites and biomass. For cross validation a partial least square (PLS) regression was performed. This method (Wold, Soft modeling by latent variables (Academic Press, London 1975)) seeks to maximize the covariance instead of the correlation between the matrices. To carry out the procedure the R function *plsr* was used. These functions are publicly available (http//www.r-project.org). All procedures were applied after missing value estimation followed by normalization of the metabolic matrix.

Relation between canonical correlation analysis (CCA), ordinary least squares (OLS) regression, partial least squares (PLS) and principal component regression (PCR):

There are several multiple regression methods, which are applied in different scientific domains like chemometrics and statistical bioinformatics. Here, the differences between and the relative merits of three of the most important methods: CCA/OLS, PLS, and PCR are explained.

The input data for each of the regression methods consist of a predictor X and a response matrix Y. While their number of rows, which is equal to the number of samples, must be the same for both, the number of columns is normally different. These regression methods can be described by the criteria they maximize (or correspondingly minimize). CCA finds the linear combinations of columns (Xw, Yv) - called canonical variates- of both matrices, which have the maximal correlation (Hotelling,H. The most predictable criterion. J Educ Psychol 26, 139-143 (1935)):

$$(\mathrm{w}, \mathrm{v}) = \arg\max_{w,v} corr(\mathrm{Xw}, \mathrm{Yv})$$

The vectors w and v are the vectors of regression coefficients. Often the response has only one column *i.e.* is represented by a vector. In this case OLS regression detects the linear combination of the predictor variables, which has the least squares difference with the response.

$$\mathrm{w} = \arg\min_{w}(\mathrm{Xw} - \mathrm{Y})^2$$

The canonical variate has the same direction as this estimation. In other words the minimum squared distances yield the maximum correlation. This method has the advantage of yielding an unbiased estimate of the regression coefficients. However, there is a trade off: The mean square error (MSE), *i.e.* the difference between true and estimated regression coefficients is often high especially as the ratio of the number of predictor variables and sample size increases. This failure is apparent in a cross validation procedure. The coefficients that maximize the correlation in the training set, often give poor results in the test set.

Therefore alternative regression methods were developed, which accept some bias in the estimation for the sake of a lower MSE. These methods effectively reduce the number of dimensions, *i.e.* the number of predictor variables. The coefficient vector w is chosen by these methods in such a way that directions, for which the spread of predictor variables is small, can be omitted.

The most extreme method is the PCR. The first step is to find a vector w, for which the variance of Xw, is maximal:

$$w = \arg\max_{w} \mathrm{var}(Xw)$$

The OLS regression is then performed on those of the new variables that have the highest variance. The disadvantage of the procedure is that the new variables are determined without considering the response. This is especially the case, if the predictor variables are largely uncorrelated.

Therefore PLS is often proposed as an alternative (Wold, Soft modelling by latent variables (Academic Press, London, 1975)). The underlying maximization principle is that of the covariance between prediction and the response: Find w such that the covariance of Xw and Y is maximal:

$$w = \arg\max_{w} \mathrm{cov}(Xw, Y) = \arg\max_{w} \mathrm{var}(Xw)\,corr(Xw, Y)$$

Since the covariance of two variables is equal to its correlation multiplied with the variance of both variables, PLS occupies an intermediate position between CCA/OLS and PCR, and it has been shown to be more appropriate for cross validation in many cases (Frank, Technometrics 35, 109-135 (1993)).

**Example 2: Biomass and metabolite profile determination of Col-0/C24 RILs**

The analyzed RIL population (Törjek, Theoretical and Applied Genetics, 2006, To be submitted) consisted of 429 lines from the reciprocal crosses Col-0xC24 (228) and C24xCol-0 (201) grown under controlled conditions in six replicated experiments. Plants harvested 15 days after sowing were used for shoot biomass determination or pooled and frozen for metabolite profiling by GC-MS. The distribution of mean biomass within the population clearly shows transgressive segregation (Fig. 1). No significant differences could be detected in marker distribution (Mantel-test, P<0.001) or biomass (t-test, P=0.238) between the two subpopulations, and the RILs were treated as one population in subsequent analyses.

**Example 3: Integrated statistical analysis of phenotypic and metabolic data**

Data for biomass and metabolite profiles were collected from 715 crosses of the RILs to parents Col-0 and C24. Concentrations could be determined for the set of 181 metabolites. The data available for these 1144 genotypes were used in pairwise correlation analysis to the untransformed data (model 1) and canonical correlation analysis (CCA) to the logarithm of the data (model 2).

Model 1 - Pairwise correlation:

The Pearson correlations between all 181 measured metabolite concentrations and the biomass were calculated. The highest absolute correlation found was: 0.23298 for a carbohydrate (Table 3). This amounts to 5.43% of variance explained by the model. The p-value was $1.55 \cdot 10^{-15}$. Further highly correlated compounds are citric acid (-0.18815 with p-value of $1.41 \cdot 10^{-10}$), ethanolamine (0.18662; $2.00 \cdot 10^{-10}$) and fructose-6-phosphate (-0.18193; $5.69 \cdot 10^{-10}$). To detect significant correlations a Bonferroni correction was performed. Thus only correlations with a p value below $0.05/181 = 2.76 \cdot 10^{-4}$ were regarded as significant.

Model 2- Canonical correlation:

The canonical correlation between the matrix of the logarithms of normalized metabolite concentrations and the logarithmic biomass vector was calculated as 0.73. This corresponds to 53.29% of variance explained by the linear combination of metabolites, almost ten times more than explained by any pairwise correlation.

To test the significance of this result, the biomass vector was permutated 50,000 times. The maximum value obtained in the permutations was 0.46. The distance between the median of the random correlations and the estimated value amounts to 17 standard deviations (Fig. 3). For normal distributions this corresponds to a p value of $4.1 \times 10^{-65}$. This

indicates that the model is statistically highly significant.

While CCA yields the maximum correlation and thus an upper limit for the true correlation, it is, dependent on the particular approach to be applied, less preferred with respect to other methods, especially partial least squares (PLS), for cross validation (Frank, Technometrics 34, 109-135 (1993)). For more details see Example 1. For the cross validation, therefore the following procedure was applied: metabolite matrix and biomass vector were divided in a training and a test set. The PLS coefficients, estimated in the training set explaining 90% of the variance of the training data, were used to predict the biomass in the test set. For a size of the training set of 1086 genotypes a median correlation between predicted and true biomass vector of 0.58 was obtained (Fig. 4). The dependence of the predictive power on the size of the training set is shown in Table 4.

The metabolites most relevant for biomass accumulation were determined by the correlation between them and the canonical variate (Razavi, BMC Medical Informatics & Decision Making 5, 29 (2005)). The first 44 metabolites with significant correlations are listed in Table 1 and displayed on biochemical pathways in Figure 5. A list of all relevant metabolites is given in Table 5.

Correlation between experimentally determined dry matter and metabolic composition

As outlined herein, pairwise correlation analysis of dry matter and single metabolites could explain a maximum of 5% of the total variance observed in biomass. These data strongly suggest that there is no single "magic" compound (at least within the metabolites analyzed) which could explain the dry matter variance in a satisfying way, a result which is not unexpected. It is in agreement with the aforementioned conclusions drawn from co-localization of biomass-QTL and metabolite-QTL.

In sharp and impressive contrast to the fact that no satisfying correlation was observed between individual metabolites and dry matter, is the finding that a combination of metabolites is highly correlated to biomass. Thus, canonical correlation analysis yielded a highly significant (the estimated p-value based on permutations is lower than $10^{-64}$) canonical correlation of 0.73 (cf. Fig. 3). Furthermore, when separating the experimental data into a training and a test set a median correlation of 0.58 between the predicted and the observed biomass was observed (cf. Fig. 4).

Inspection of the metabolites highly ranked in the canonical correlation analysis and thus representing the main drivers of the correlation shows that central metabolism derived metabolites are strongly represented such as the sugar phosphates glucose-6-phosphate and fructose-6-phosphate, members of the TCA cycle such as succinate, citrate and malate or sucrose. Other metabolites such as glycerol-3-phosphate, ethanolamine or sinapine play a major role in membrane/phospholipid biosynthesis. The anti-oxidant ascorbic acid (vitamin C) belongs to the highly ranked metabolites in CAA, and its only QTL co-localizes with the biomass QTL at 1/88. Ascorbic acid has also been implicated in cell division (Liso, Exp Cell Res 150, 314-320 (1984)) and plant growth regulation via its role as enzyme co-factor (Smirnoff, Curr Opin Plant Biol 3, 229-235 (2000)). Glutamine as a central metabolite in nitrogen assimilation and amino donor is also found amongst the most important metabolites. This is contrasted by the fact that nearly all other amino acids analyzed are of rather low contribution based on the CCA analysis. Further highly ranking metabolites can at first approximation be assigned to general stress metabolites such as the polyamines putrescine, spermidine and ornithine. Thus, a link between the metabolites ranked high in the CCA analysis and biomass accumulation is plausible since central metabolism and stress response are of utmost importance to plant growth and thus biomass.

Another surprising result originating from the CCA analysis is that both positive and negative correlations are found between metabolites and biomass. A closer look at the 44 metabolites most important in the CCA ranking reveals some interesting patterns. Thus, the large majority of known metabolites displaying a negative correlation to the biomass vector are derived from central metabolic pathways such as sucrose, glucose- and fructose-6-phosphate, several members of the TCA cycle such as citric acid, succinate or malic acid as well as the amino acids glutamine and phenylalanine. On the other hand, amongst the positively correlated metabolites are a large fraction of unknown chemical structure as well as some metabolites discussed in stress response such as nicotinic acid (Hageman, Mutat Res-Fund Mol M 475, 45-56 (2001)) or putrescine (Tkachenko, Microbiology 70, 422-428 (2001)), or the stress metabolite trehalose discussed in connection with drought resistance (Garg, P Ntal Acad Sci UUSA 99, 15898-15903 (2002) (Jang, Plant Physiol 131, 516-524 (2003)). The negative correlation suggests that pool sizes of these metabolites are reduced to a minimally allowed value when maximal growth occurs. It is conceivable that this involves mostly metabolites providing the major building blocks for growth such as the central metabolites mentioned. A similar conclusion of metabolism driven by growth has been derived from a study of the relationship between tomato fruit size and metabolites (Schauer, Nat Biotechnol 24, 447-454 (2006)). In this scenario the positively correlated metabolites have a role in defending the plant against abiotic and biotic stress and it is comprehensible that a higher concentration of these metabolites correlates with a better armed plant. An alternative/complementary hypothesis regards metabolites not primarily as chemicals for growth and defense but rather as signals. Under this assumption positively correlated metabolites are positive signals regulating plant growth and the contrary would be true for negatively correlated metabolites. As a further consequence one has to assume proteins sensing these molecules which either act as repressors or activators of growth. In the context of signal molecules the large number of positively correlated compounds of as yet unknown structure is worth noting and stresses the need for identification of their chemical nature. They might constitute unusual products of metabolic side reactions that are derived from primary metabolites generated for signaling purposes and which can move to sites of perception without further conversion along the major metabolic reactions or transport pathways. Further studies querying some testable predictions from such models (e.g. the presence of receptors/sensors or the elicitation of specific responses in case of signaling metabolites) can further validate these models.

Irrespective of the underlying mechanisms it is clear from the preceding descriptions that the metabolic composition is highly correlated to biomass and can actually be used to determine (the potential for) biomass production/growth. This was shown in a convincing way by the result of the meta QTL search using the predicted biomass value as a new trait (cf. Fig. 6).

**Example 4: QTL analyses: Identification of QTL for shoot dry biomass and metabolites**

The shoot biomass and metabolite data were used to map QTL based on a linkage map of 105 markers established for the Col-0/C24 RIL population (Törjék Theoretical & Applied Genetics, 2006, to be submitted) by application of the two software packages PLABQTL (Utz, J QTL2 (1996)) and QTL-Cartographer (Basten, Computing Strategies and Software 22, 65-66 (1994)).

QTL analysis of shoot dry biomass:

A complete list and description of QTL detected for shoot biomass is given in Table 2. The explained phenotypic (denoted by $R^2$) and genotypic variation is obtained from the final simultaneous fit of all putative QTL in PLABQTL. For biomass, six QTL explain 18.5 $\pm$ 3.4% of the phenotypic and 26.8 $\pm$ 4.9% of the genotypic variation. Individual QTL contributions range from 1.5 to 6.0% of the total variance. The mean $R^2$ after cross-validation was 16.01 % in the calibration and 8.92% in the validation, for a mean number of six QTL.

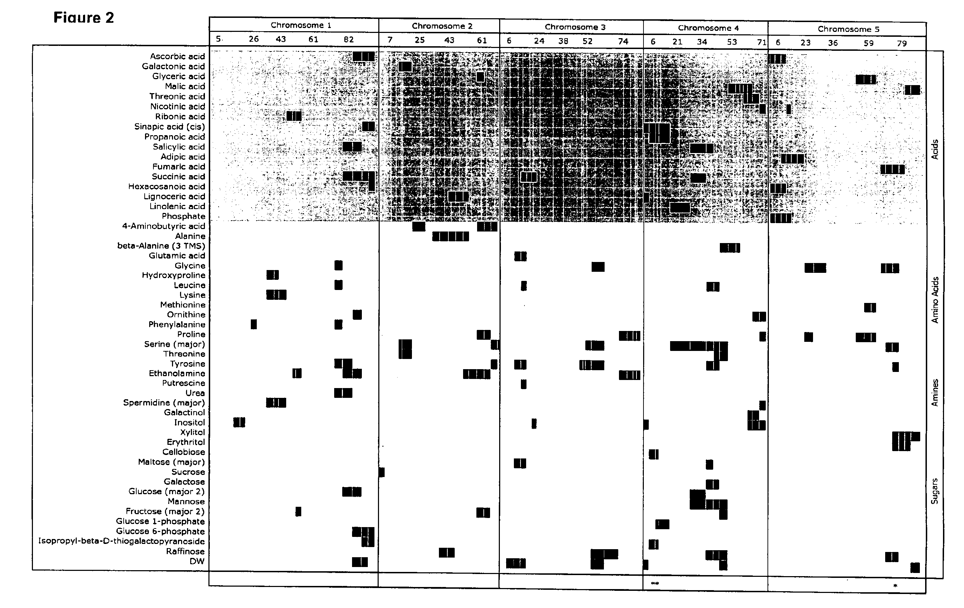

As described herein (cf. Fig. 2), the variance of the RIL population analyzed allowed the successful identification of QTL for shoot biomass as well as for a number of metabolites. The results for biomass are similar to data described for other *Arabidopsis* RIL populations that were used to detect QTL for aerial / shoot mass with up to eight QTL detected (El-Lithy, Plant Physiol 135, 444-458 (2004)), (Loudet, O., Plant Physiol 131, 345-358 (2003)), (Rauh, Theor Appl Genet 104, 743-50 (2002)),(Ungerer, Evolution 57, 2531-2539 (2003)). In a comparable study of biomass at an early developmental stage in *Aegilops tauschii* Steege, (Plant Physiol 139, 1078-1094 (2005)), only two putative QTL could be detected. In seedling stage corn, three QTL for shoot dry weight each explaining 11 to 15% of phenotypic variance were detected in a F2:F3 population of 226 families (Jompuk, J Exp Bot 56, 1153-1163 (2005)). Further biomass QTL analyses *e.g.* of poplar (Wullschleger, Can J Forest Res 35, 1779-1789 (2005)), rice (Hittalmani, Euphytica 125, 207-214 (2002)), Li, Plant Sci 170, 911-917 (2006)), and *Miscanthus sinensis* (Atienza, Euphytica 132, 353-361 (2003)) each revealed a limited number of QTL usually with a restricted fraction of the phenotypic variance explained. Even in a very large QTL mapping experiment in corn (Schön, Genetics 167, 485-498 (2004)) with more than 30 identified growth related QTL, only about 50% of the genetic variance were explained. The effects of individual QTL on the phenotypic variance were generally small. Thus, the individual contribution of shoot biomass QTL in the Arabidopsis RIL population analyzed here is very similar to the situation described for other species including crops such as corn despite the fact that corn has been selected for yield for several decades.

QTL analysis of metabolites:

Samples taken from 369 RILs were analyzed for their metabolic composition. A total of 181 compounds could be detected in more than 85% of all samples and only those metabolites were taken into further consideration. For 95 of these compounds the chemical nature is known.

In total 228 metabolic QTL for 119 metabolites were found. For 63 metabolites only one QTL was identified whereas a maximum of seven QTL was found for tyrosine. The QTL are distributed unequally over marker positions indicating 'hot spots' (Fig. 2) and empty regions (no metabolic QTL at 10 marker positions). The contribution of individual QTL to the phenotypic variation varied between 1.9 and 50.9% (cellobiose). A comparative overview of QTL for known metabolites and biomass is presented in Fig. 2.

Preliminary analysis of detected metabolic QTL with respect to underlying biochemical pathways show that it is possible to identify candidate genes even at this rather low mapping resolution. For example, inspection of the available information on pathways involving myo-inositol suggested candidate genes for all identified QTL (Fig. 7).

As to metabolic QTL, it was succeeded to identify for more than half of the compounds analyzed at least one QTL (181 compounds were analyzed, at least one QTL was identified for 119 compounds) with the contribution of individual QTL to the total phenotypic variance ranging from 1.9 to more than 50%. Although the 181 compounds analyzed in the metabolic profiling experiments represent only a portion of the total metabolites present in a given cell it was assumed that this subgroup is representative for the total entity of metabolites, also demonstrated by an untargeted metabolomics approach using anonymous mass peaks for genetic and QTL analyses in *Arabidopsis* (Keurentjes, Nat Genet 1815, (2006)). Under this assumption a few interesting features of the metabolic QTL are discussed below.

First, the chromosomal distribution of the total 228 metabolic QTL differs in a statistically significant manner from a random distribution displaying two significant hot spots. Second, a number of QTL which are shared between several metabolites were identified. Two situations can be distinguished:

The metabolites sharing a QTL are derived from the same biochemical pathway or from related pathways as observed for serine/glycine (position 3/57). This makes this QTL a candidate for a pathway QTL which could be either a gene controlling the formation of a rate-limiting precursor or a higher hierarchy controller of the entire pathway such as a transcription factor. In other cases (cf. position 3/14) metabolites with common QTL are derived from widely divergent pathways, which could be due to a major controller of several pathways or a small molecule produced in one pathway and controlling the other pathway. At this point the limited genetic resolution does not allow to exclude the much more trivial possibility of the shared genomic regions actually being composed of several different linked QTL. This possibility can only be excluded by increasing the precision of the map position via fine mapping *e.g.* using suitable genetic substitution lines and ultimately by identification of the polymorphism(s) responsible for the variation of the metabolites.

Irrespective of the fact that the resolution is still limited, an analysis of the metabolic QTL with respect to candidate genes as derived from known biochemical pathways is surprisingly fruitful. Inspection of the myo-inositol pathway allows the identification of candidate genes within the region of all myo-inositol QTL identified (cf. Fig. 7).

**Example 5: Co-localization of QTL for shoot dry biomass and correlated metabolites**

All biomass QTL were co-localized with five to eleven metabolite QTL and two of them co-locate with significantly more metabolite QTL than expected, if the latter would be distributed randomly on the chromosomes. This observation indicates that variation in growth is related to changes in metabolite levels. If changes in growth e.g. due to modulation via a growth regulator encoded at the QTL would result in altered metabolism, this should cause changes in similar sets of metabolites. The observation that QTL of very different sets of metabolites co-locate with growth-QTL in different areas, thus may indicate that at these positions changes in (different) metabolites trigger the enhancement or the reduction of growth rather than the other way around.

The small fraction of the phenotypic variance (maximum 6%) explained by a single growth QTL leads to the interpretation that variation of individual metabolites has only weak effects on overall growth. In other words, major variation in growth apparently is only brought about by the joint action and interaction of very many genetic (and environmental) factors, which individually have very weak effects (e.g. via effects on the level of an individual metabolite) and of which only few can be singled out as detectable QTL. This interpretation is supported by the conclusions drawn from the analysis of two growth rate QTL found in a 210 kb interval in *Arabidopsis thaliana* that both showed epistasis and indicated that complex traits such as growth rate are highly polygenic with numerous interactions among the involved factors (Kroymann, Nature 435, 95-98 (2005)).

By analyzing the metabolites representing at least in their combination the dry matter of the plant insight was gained into possible pathways involved in the biomass QTL using co-localized metabolic QTL. Metabolites from both groups - high and low ranked in CCA - have QTL in the same regions as biomass. To further investigate this interesting finding we performed a meta QTL search using the predicted biomass vector (canonical variate) as a new trait. The LOD curves for both traits overlap in 10 of 17 peaks in PLABQTL and 6 of 12 in QTL-Cartographer (Fig. 6). Here one should note that the canonical variate was computed on the combined data set (RIL plus crosses), while the QTL mapping was applied on the RIL population only. At positions 88 cM on chromosome 1 (denoted as 1/88) and 4/0, the respective biomass QTL co-locate with significantly more metabolite QTL than expected in a random distribution (Fig. 2 and Table 2).

The agreement between experimental and predicted biomass QTL is strikingly good - especially regarding the fact that the QTL analysis was only done on a subset of the data used in the CCA and that the median correlation of the predicted and the true biomass values was 0.58. Thus three out of six biomass QTL were validated with this approach. The failure to reproduce the dry matter QTL on chromosome 4 is in further agreement with the finding that the metabolite QTL co-located in these regions are largely of metabolites with low ranking in the CCA. The meta QTL search also provides indications for further biomass QTL, which were below the significant threshold in the original analysis.

The present invention refers to the following tables:

Table 1: List of the 44 most relevant metabolites ranked according to the strength of the correlation with respect to the canonical variate. Given are the correlation (COR) and the corresponding p-value (PV).

| METABOLITE | COR | PV |
|---|---|---|
| unknown_038* | 0,37833 | 0,00E+00 |
| unknown_035* | 0,31038 | 0,00E+00 |
| Ethanolamine | 0,30515 | 0,00E+00 |
| unknown_086* | -0,27201 | 7,45E-21 |
| Fructose 6-phosphate | -0,24840 | 1,51 E-17 |
| Citric acid | -0,24195 | 1,06E-16 |
| unknown_078* | 0,23882 | 2,22E-16 |
| unknown_061* | 0,22967 | 3,77E-15 |
| Glutamine | -0,22258 | 2,62E-14 |
| Glycerol-3-phosphate | -0,22088 | 4,16E-14 |
| Sinapic acid (cis) | -0,21462 | 2,19E-13 |
| Raffinose | -0,20030 | 8,09E-12 |
| Ornithine | 0,19723 | 1,70E-11 |
| Putrescine | 0,19409 | 3,57E-11 |
| unknown_051 | 0,19398 | 3,68E-11 |
| Glucose 6-phosphate | -0,18921 | 1,11 E-10 |
| Spermidine (major) | 0,18798 | 1,47E-10 |
| unknown_048 | -0,18557 | 2,54E-10 |
| Sinapic acid (trans) | -0,17943 | 9,84E-10 |
| Sucrose | -0,17937 | 9,98E-10 |
| unknown_074 | 0,17879 | 1,13E-09 |
| Citramalic acid | -0,17388 | 3,22E-09 |
| Ascorbic acid | -0,16929 | 8,34E-09 |
| Tyrosine | -0,15838 | 7,25E-08 |
| unknown_062 | -0,15359 | 1,79E-07 |
| Succinic acid | -0,15190 | 2,44E-07 |
| unknown_071* | -0,14931 | 3,92E-07 |
| Malic acid | -0,14215 | 1,39E-06 |
| Trehalose | 0,13961 | 2,14E-06 |
| unknown_033 | 0,13924 | 2,28E-06 |
| unknown_091 | 0,13649 | 3,60E-06 |
| unknown_060 | 0,12791 | 1,43E-05 |
| Nicotinic acid | 0,12497 | 2,25E-05 |
| unknown_043 | 0,12443 | 2,44E-05 |
| unknown_054 | -0,12395 | 2,62E-05 |
| unknown_063 | 0,12240 | 3,31 E-05 |
| unknown_088 | -0,11951 | 5,07E-05 |
| unknown_011 | 0,11505 | 9,62E-05 |
| unknown_084 | 0,11208 | 1,46E-04 |
| Maleic acid | -0,11167 | 1,54E-04 |
| Phenylalanine | -0,11090 | 1,71 E-04 |
| Salicylic acid | -0,11060 | 1,78E-04 |
| unknown_005 | -0,10851 | 2,36E-04 |
| unknown_056 | 0,10746 | 2,71 E-04 |

* MassSpectrum indicates following chemical classes for these unknown compounds: 038 - sugar; 035 - glucopyranoside; 086 - lactobionic acid; 078 - pyranoside; 061 - polyol; 071 - sugar phosphate

Table 2: QTL for shoot dry weight and metabolites detected via PLABQTL and QTL Cartographer, which showed a 5% significance in one program and at least a 25% significance in the other program.

Position of the QTL is given by chromosome / position on chromosome in cM. Left_Mark is the closest marker to the left of the QTL. Supp.IV indicates the confidence interval of the QTL, in cM on the respective chromosome. $R^2$ (%) corresponds to the percentage phenotypic variation explained by the QTL. The favorable allele is indicated in the last column.

| Trait | Position | Left_Mark | Supp.IV | $R^2$ (%) | fav.allele |
|---|---|---|---|---|---|
| DW | 1/88 | I.22 | 82-94 | 1.53 | Col-0 |
| DW | 3/13 | III.50 | 10-18 | 5.96 | C24 |
| DW | 3/59 | III.63 | 52-66 | 3.45 | Col-0 |
| DW | 4/0 | IV.70 | 0-2 | 5.27 | Col-0 |
| DW | 4/47 | IV.79 | 44-54 | 3.96 | Col-0 |
| DW | 5/86 | V.110 | 82-90 | 2.86 | C24 |
| 4-Aminobutyric acid | 2 / 22 | 11.32 | 16-25 | 4.38 | C24 |
| 4-Aminobutyric acid | 2/61 | 11.42 | 57-71 | 3.61 | C24 |
| Adipic acid | 5/15 | V.90 | 8-19 | 3.28 | C24 |
| Alanine | 2/43 | II.36 | 32-54 | 3.06 | C24 |
| beta-Alanine (3 TMS) | 4/51 | IV.79 | 49-56 | 6.22 | Col-0 |
| Cellobiose | 4/6 | IV.71 | 5-6 | 52.10 | C24 |
| Erythritol | 5/77 | V.108 | 75-82 | 5.74 | C24 |
| Ethanolamine | 1/52 | I.14 | 50-52 | 8.69 | Col-0 |
| Ethanolamine | 1/84 | I.22 | 82-90 | 5.34 | Col-0 |
| Ethanolamine | 2/59 | II.41 | 52-66 | 3.34 | Col-0 |
| Ethanolamine | 3/84 | III.65 | 74-84 | 2.98 | Col-0 |
| Fructose (major 2) | 1/52 | I.15 | 52-56 | 3.12 | C24 |
| Fructose (major 2) | 4/47 | IV.78 | 45-51 | 3.39 | Col-0 |
| Fumaric acid | 5/71 | V.106 | 67-81 | 3.61 | Col-0 |
| Galactose | 4/42 | IV.76 | 38 - 42 | 2.64 | Col-0 |
| Glucose (major 2) | 1/84 | 1.21 | 78 - 92 | 3.67 | C24 |
| Glucose (major 2) | 4/34 | IV.75 | 29 - 38 | 4.35 | Col-0 |
| Glucose 1-phosphate | 4/8 | IV.73 | 8 - 12 | 10.73 | C24 |
| Glucose 6-phosphate | 1/100 | 1.23 | 88 - 100 | 3.63 | C24 |
| Glyceric acid | 2/61 | II.42 | 57 - 63 | 3.01 | C24 |
| Glyceric acid | 5/56 | V.101 | 51- 62 | 3.55 | C24 |
| Glycine | 1/76 | I.20 | 74-80 | 4.08 | C24 |
| Glycine | 3/56 | III.61 | 54-59 | 8.05 | C24 |
| Glycine | 5/30 | V.95 | 23-34 | 3.10 | Col-0 |
| Glycine | 5/69 | V.106 | 67-75 | 7.03 | C24 |
| Hexacosanoic acid | 1/96 | 1.24 | 95-100 | 2.93 | C24 |
| Hexacosanoic acid | 4/2 | IV.70 | 2-4 | 14.73 | C24 |
| Hexacosanoic acid | 5/8 | V.89 | 2-11 | 3.05 | Col-0 |
| Hydroxyproline | 1/37 | I.9 | 34-39 | 5.03 | Col-0 |
| Inositol | 1/18 | I.4 | 14-20 | 6.52 | C24 |

(continued)

Position of the QTL is given by chromosome / position on chromosome in cM. Left_Mark is the closest marker to the left of the QTL. Supp.IV indicates the confidence interval of the QTL, in cM on the respective chromosome. $R^2$ (%) corresponds to the percentage phenotypic variation explained by the QTL. The favorable allele is indicated in the last column.

| Trait | Position | Left_Mark | Supp.IV | $R^2$ (%) | fav.allele |
|---|---|---|---|---|---|
| Inositol | 3/20 | III.53 | 20 - 22 | 2.75 | Col-0 |
| Inositol | 4/0 | IV.70 | 0 - 2 | 12.68 | Col-0 |
| Inositol | 4/70 | IV.85 | 68 - 73 | 8.46 | Col-0 |
| Isopropyl-beta-D-thiogalactopyranoside | 1/92 | I.23 | 90 - 100 | 3.84 | C24 |
| Isopropyl-beta-D-thiogalactopyranoside | 4/8 | IV.70 | 4 - 8 | 7.97 | C24 |
| Leucine | 4/43 | IV.76 | 38 - 45 | 4.67 | Col-0 |
| Lignoceric acid | 2/48 | 11.39 | 43 - 52 | 2.88 | C24 |
| Lignoceric acid | 4/2 | IV.70 | 0 - 4 | 15.51 | C24 |
| Linolenic acid | 4/18 | IV.74 | 12 - 29 | 5.01 | C24 |
| Lysine | 1/39 | 1.9 | 36 - 45 | 2.80 | Col-0 |
| Malic acid | 4/55 | IV.79 | 49 - 65 | 4.23 | Col-0 |
| Malic acid | 5/82 | V.109 | 81 - 90 | 3.03 | Col-0 |
| Maltose (major) | 4/38 | IV.76 | 36-42 | 9.93 | Col-0 |
| Nicotinic acid | 4/71 | IV.85 | 68 - 73 | 3.10 | Col-0 |
| Nicotinic acid | 5/8 | V.90 | 8-21 | 9.58 | Col-0 |
| Ornithine | 1/88 | 1.22 | 86-92 | 4.13 | Col-0 |
| Ornithine | 4/71 1 | IV.84 | 65-73 | 3.69 | C24 |
| Phenylalanine | 1 / 26 | I.7 | 24-28 | 3.26 | Col-0 |
| Phenylalanine | 1/76 | 1.20 | 74-80 | 3.06 | C24 |
| Proline | 2/59 | 11.42 | 57-61 | 9.61 | C24 |
| Proline | 3/82 | III.64 | 72-84 | 4.00 | Col-0 |
| Proline | 4/71 | IV.85 | 70-71 | 7.92 | C24 |
| Proline | 5 / 23 | V.94 | 21 - 25 | 6.74 | Col-0 |
| Propanoic acid | 4/8 | IV.70 | 2-12 | 8.00 | Col-0 |
| Putrescine | 3/24 | III.53 | 20-32 | 3.09 | C24 |
| Raffinose | 2/41 | II.37 | 35-47 | 5.03 | C24 |
| Raffinose | 3/68 | III.63 | 57-72 | 4.70 | C24 |
| Raffinose | 4/43 | IV.77 | 40-49 | 4.17 | Col-0 |
| Raffinose | 5/73 | V.107 | 71-73 | 6.39 | C24 |
| Salicylic acid | 1/80 | I.21 | 78 - 90 | 7.95 | C24 |
| Salicylic acid | 4/38 | IV.76 | 36 - 38 | 12.27 | Col-0 |
| Serine (major) | 2/13 | II.29 | 12 - 24 | 5.11 | C24 |
| Serine (major) | 2/69 | II.44 | 66 - 71 | 4.31 | C24 |
| Serine (major) | 3/54 | III.61 | 52 - 57 | 6.88 | C24 |
| Serine (major) | 4/43 | IV.75 | 21 - 51 | 2.67 | Col-0 |
| Serine (major) | 5/71 | V.106 | 69 - 75 | 3.76 | C24 |
| Sinapic acid (cis) | 1/96 | I.23 | 92 - 100 | 3.05 | C24 |
| Sinapic acid (cis) | 4/4 | IV.70 | 0 - 14 | 5.69 | C24 |
| Spermidine (major) | 4/71 | IV.85 | 70 - 73 | 5.72 | C24 |
| Succinic acid | 1/90 | I.21 | 78 - 98 | 4.54 | C24 |
| Succinic acid | 3/14 | III.50 | 13 - 22 | 3.66 | C24 |
| Succinic acid | 4/31 | IV.75 | 25 - 38 | 7.12 | Col-0 |
| Sucrose | 2/0 | II.27 | 0 - 6 | 4.03 | C24 |

(continued)

Position of the QTL is given by chromosome / position on chromosome in cM. Left_Mark is the closest marker to the left of the QTL. Supp.IV indicates the confidence interval of the QTL, in cM on the respective chromosome. $R^2$ (%) corresponds to the percentage phenotypic variation explained by the QTL. The favorable allele is indicated in the last column.

| Trait | Position | Left_Mark | Supp.IV | $R^2$ (%) | fav.allele |
|---|---|---|---|---|---|
| Threonic acid | 4/63 | IV.83 | 60 - 70 | 3.69 | Col-0 |
| Threonine | 2/13 | II.29 | 12 - 20 | 5.55 | C24 |
| Threonine | 4/49 | IV.78 | 43 - 51 | 4.11 | Col-0 |
| Tyrosine | 1/78 | I.20 | 74 - 84 | 3.93 | C24 |
| Tyrosine | 2/71 | II.44 | 66 - 71 | 2.62 | C24 |
| Tyrosine | 3/14 | III.49 | 10 - 16 | 3.23 | C24 |
| Tyrosine | 3/50 | III.57 | 48-63 | 4.18 | C24 |
| Tyrosine | 4/38 | IV.76 | 36-45 | 7.91 | Col-0 |
| Tyrosine | 5/74 | V.107 | 72-76 | 0.00 | C24 |
| unknown_003 | 3/54 | III.61 | 52-61 | 5.41 | C24 |
| unknown_003 | 5/9 | V.90 | 8-15 | 5.86 | C24 |
| unknown_004 | 1/9 | I.2 | 7-11 | 2.58 | Col-0 |
| unknown_005 | 3/34 | III.54 | 30-36 | 0.00 | C24 |
| unknown_006 | 4/60 | IV.83 | 60-62 | 0.00 | C24 |
| unknown_007 | 4/4 | IV.70 | 2-6 | 6.73 | Col-0 |
| unknown_008 | 1/16 | I.5 | 16-18 | 0.00 | Col-0 |
| unknown_008 | 1 / 28 | I.8 | 26 - 28 | 0.00 | C24 |
| unknown_009 | 1/74 | I.19 | 69 - 78 | 3.52 | Col-0 |
| unknown_009 | 4/2 | IV.70 | 0 - 4 | 19.28 | Col-0 |
| unknown_012 | 2/13 | II.28 | 9 - 18 | 3.43 | C24 |
| unknown_013 | 1/64 | I.17 | 62 - 66 | 0.00 | C24 |
| unknown_013 | 4/6 | IV.70 | 2 - 12 | 3.57 | Col-0 |
| unknown_018 | 1/94 | 1.23 | 92 - 96 | 0.00 | C24 |
| unknown_020 | 5/92 | V.110 | 82 - 92 | 3.82 | Col-0 |
| unknown_021 | 4/6 | IV.70 | 4 - 6 | 0.00 | Col-0 |
| unknown_022 | 1/39 | I.11 | 39 - 45 | 2.79 | C24 |
| unknown_022 | 1/76 | 1.20 | 74 - 84 | 3.88 | Col-0 |
| unknown_023 | 5/86 | V.110 | 84 - 92 | 2.76 | Col-0 |
| unknown_025 | 5/16 | V.92 | 14 - 18 | 0.00 | Col-0 |
| unknown_026 | 4/65 | IV.82 | 58 - 73 | 3.43 | Col-0 |
| unknown_028 | 1/94 | 1.23 | 92 - 94 | 0.00 | C24 |
| unknown_028 | 3/50 | III.60 | 50 - 51 | 0.00 | C24 |
| unknown_029 | 1/94 | I.23 | 92 - 94 | 0.00 | C24 |
| unknown_030 | 1/47 | I.12 | 45 - 48 | 5.17 | C24 |
| unknown_030 | 1/88 | I.21 | 76 - 90 | 5.05 | Col-0 |
| unknown_031 | 4/6 | IV.71 | 6-8 | 35.29 | Col-0 |
| unknown_033 | 1 / 26 | I.7 | 24-26 | 0.00 | Col-0 |
| unknown_034 | 3/82 | III.68 | 82-84 | 0.00 | Col-0 |
| unknown_035 | 1/10 | I.3 | 10-11 | 0.00 | C24 |
| unknown_035 | 2/2 | II.27 | 0-7 | 4.56 | Col-0 |
| unknown_035 | 2/32 | II.36 | 31-33 | 0.00 | C24 |
| unknown_035 | 2/64 | II.42 | 57-66 | 2.88 | Col-0 |
| unknown_035 | 4/6 | IV.70 | 2-8 | 5.74 | C24 |
| unknown_035 | 4/20 | IV.74 | 16-20 | 0.00 | C24 |
| unknown_037 | 4/6 | IV.70 | 2-8 | 4.38 | Col-0 |

(continued)

Position of the QTL is given by chromosome / position on chromosome in cM. Left_Mark is the closest marker to the left of the QTL. Supp.IV indicates the confidence interval of the QTL, in cM on the respective chromosome. $R^2$ (%) corresponds to the percentage phenotypic variation explained by the QTL. The favorable allele is indicated in the last column.

| Trait | Position | Left_Mark | Supp.IV | $R^2$ (%) | fav.allele |
|---|---|---|---|---|---|
| unknown_038 | 1/9 | I.1 | 0-18 | 3.84 | C24 |
| unknown_038 | 5/80 | V.109 | 80-84 | 0.00 | C24 |
| unknown_039 | 4/8 | IV.71 | 6-8 | 4.80 | C24 |
| unknown_041 | 3/50 | III.60 | 50-51 | 0.00 | C24 |
| unknown_041 | 5/80 | V.108 | 78-80 | 0.00 | C24 |
| unknown_045 | 4/8 | IV.73 | 8-9 | 0.00 | C24 |
| unknown_047 | 3/4 | III.48 | 2-4 | 0.00 | Col-0 |
| unknown_048 | 1/100 | I.25 | 98-100 | 0.00 | C24 |
| unknown_048 | 3/80 | III.66 | 79-80 | 0.00 | C24 |
| unknown_048 | 4/10 | IV.74 | 10-12 | 0.00 | Col-0 |
| unknown_051 | 1 / 26 | I.7 | 24-28 | 5.32 | Col-0 |
| unknown_051 | 1/34 | I.5 | 18-36 | 4.60 | Col-0 |
| unknown_051 | 4/2 | IV.70 | 2-4 | 0.00 | Col-0 |
| unknown_051 | 5/92 | V.111 | 88-92 | 0.00 | C24 |
| unknown_052 | 3/72 | III.63 | 67-76 | 5.99 | Col-0 |
| unknown_052 | 4/4 | IV.70 | 2-10 | 21.87 | C24 |
| unknown_052 | 4/8 | IV.73 | 7-8 | 21.72 | C24 |
| unknown_052 | 5/12 | V.92 | 12-14 | 0.00 | C24 |
| unknown_052 | 5/21 | V.94 | 21-27 | 15.85 | C24 |
| unknown_052 | 5/77 | V.107 | 73-81 | 3.30 | C24 |
| unknown_053 | 5/92 | V.110 | 84-92 | 3.11 | C24 |
| unknown_054 | 1 / 20 | I.6 | 20-24 | 0.00 | C24 |
| unknown_055 | 3/20 | III.53 | 20-22 | 4.87 | Col-0 |
| unknown_055 | 3/84 | III.68 | 82-84 | 4.36 | Col-0 |
| unknown_055 | 5/90 | V.111 | 88-92 | 0.00 | C24 |
| unknown_056 | 1/93 | I.23 | 90-100 | 2.71 | C24 |
| unknown_056 | 4/8 | IV.73 | 7-8 | 15.13 | C24 |
| unknown_056 | 5/23 | V.95 | 23-25 | 13.74 | C24 |
| unknown_056 | 5/77 | V.108 | 75-81 | 6.85 | C24 |
| unknown_058 | 1 / 62 | 1.17 | 62 - 64 | 0.00 | C24 |
| unknown_058 | 4 / 8 | IV.73 | 8 - 9 | 15.32 | C24 |
| unknown_058 | 5/23 | V.95 | 23 - 25 | 11.90 | C24 |
| unknown_058 | 5 / 77 | V.107 | 73 - 84 | 5.64 | C24 |
| unknown_060 | 3 / 70 | III.63 | 63 - 76 | 2.17 | Col-0 |
| unknown_060 | 4 / 4 | IV.70 | 4 - 8 | 27.44 | C24 |
| unknown_060 | 5 / 12 | V.91 | 10 - 12 | 0.00 | C24 |
| unknown_060 | 5/25 | V.95 | 23 - 25 | 19.20 | C24 |
| unknown_060 | 5 / 77 | V.107 | 73 - 81 | 3.64 | C24 |
| unknown_061 | 5/40 | V.97 | 40 - 41 1 | 0.00 | C24 |
| unknown_062 | 1/26 | 1.8 | 24 - 26 | 0.00 | Col-0 |
| unknown_062 | 1/34 | 1.9 | 32 - 36 | 14.65 | C24 |
| unknown_062 | 4/2 | IV.70 | 0-2 | 0.00 | Col-0 |
| unknown_063 | 1/86 | I.21 | 80 - 98 | 2.76 | C24 |
| unknown_063 | 4/8 | IV.73 | 8-9 | 17.15 | C24 |
| unknown_063 | 5 / 23 | V.95 | 23 - 25 | 12.26 | C24 |

(continued)

Position of the QTL is given by chromosome / position on chromosome in cM. Left_Mark is the closest marker to the left of the QTL. Supp.IV indicates the confidence interval of the QTL, in cM on the respective chromosome. $R^2$ (%) corresponds to the percentage phenotypic variation explained by the QTL. The favorable allele is indicated in the last column.

| Trait | Position | Left_Mark | Supp.IV | $R^2$ (%) | fav.allele |
|---|---|---|---|---|---|
| unknown_063 | 5/75 | V.107 | 73 - 84 | 2.26 | C24 |
| unknown_064 | 1 / 26 | I.8 | 24 - 26 | 0.00 | Col-0 |
| unknown_064 | 1/36 | 1.9 | 34 - 37 | 21.05 | C24 |
| unknown_064 | 3/74 | III.64 | 72 - 76 | 3.49 | Col-0 |
| unknown_064 | 4/2 | IV.70 | 0-4 | 8.27 | Col-0 |
| unknown_065 | 1 / 32 | 1.9 | 30-34 | 0.00 | C24 |
| unknown_067 | 1 / 50 | 1.14 | 49 - 51 | 0.00 | Col-0 |
| unknown_067 | 1 / 76 | I.21 | 73 - 76 | 0.00 | C24 |
| unknown_067 | 4/16 | IV.74 | 12-28 | 0.00 | C24 |
| unknown_068 | 1 / 96 | 1.23 | 92 - 100 | 3.77 | C24 |
| unknown_068 | 4 / 10 | IV.74 | 9 - 10 | 0.00 | C24 |
| unknown_068 | 4 / 66 | IV.84 | 64-68 | 0.00 | Col-0 |
| unknown_068 | 5 / 8 | V.89 | 2 - 15 | 3.89 | Col-0 |
| unknown_068 | 5/58 | V.102 | 56-62 | 3.64 | Col-0 |
| unknown_069 | 3/30 | III.54 | 26-32 | 3.87 | C24 |
| unknown_070 | 1/84 | I.22 | 82-86 | 0.00 | Col-0 |
| unknown_070 | 4/30 | IV.75 | 26-34 | 0.00 | C24 |
| unknown_070 | 5/73 | V.107 | 71-82 | 3.66 | C24 |
| unknown_071 | 2/44 | II.38 | 40-46 | 0.00 | C24 |
| unknown_072 | 2/4 | II.27 | 2-6 | 15.01 | Col-0 |
| unknown_072 | 3/82 | III.68 | 82-84 | 0.00 | Col-0 |
| unknown_073 | 1/100 | I.24 | 95-100 | 3.19 | C24 |
| unknown_073 | 2 / 7 | II.27 | 2-9 | 4.17 | Col-0 |
| unknown_074 | 1/72 | 1.19 | 69-74 | 9.30 | Col-0 |
| unknown_074 | 4/6 | IV.70 | 4-6 | 15.77 | C24 |
| unknown_074 | 4/70 | IV.85 | 70-72 | 0.00 | Col-0 |
| unknown_075 | 1/26 | 1.8 | 25-27 | 0.00 | Col-0 |
| unknown_075 | 1/36 | 1.9 | 34-37 | 27.62 | C24 |
| unknown_075 | 1/71 | I.19 | 69-74 | 2.73 | Col-0 |
| unknown_075 | 1/100 | I.25 | 98-100 | 0.00 | C24 |
| unknown_075 | 3/74 | III.64 | 70-78 | 3.41 | Col-0 |
| unknown_075 | 3/82 | III.68 | 82-84 | 3.29 | Col-0 |
| unknown_075 | 4/2 | IV.70 | 0-4 | 10.60 | Col-0 |
| unknown_075 | 5/66 | V.105 | 66-68 | 0.00 | Col-0 |
| unknown_076 | 4/63 | IV.83 | 62-68 | 3.42 | Col-0 |
| unknown_076 | 5/78 | V.108 | 78-80 | 0.00 | C24 |
| unknown_077 | 5/73 | V.106 | 69-77 | 4.64 | C24 |
| unknown_078 | 3/26 | III.54 | 24-32 | 6.98 | C24 |
| unknown_078 | 5/72 | V.106 | 70-74 | 0.00 | C24 |
| unknown_079 | 2/41 | II.38 | 40-42 | 26.44 | Col-0 |
| unknown_079 | 4/8 | IV.73 | 8-9 | 10.73 | C24 |
| unknown_081 | 4/8 | IV.73 | 7-8 | 0.00 | C24 |
| unknown_083 | 5/38 | V.96 | 36-38 | 0.00 | Col-0 |
| unknown_084 | 1/34 | I.9 | 32-37 | 4.44 | C24 |
| unknown_084 | 1/72 | I.19 | 71-72 | 44.62 | C24 |

(continued)

Position of the QTL is given by chromosome / position on chromosome in cM. Left_Mark is the closest marker to the left of the QTL. Supp.IV indicates the confidence interval of the QTL, in cM on the respective chromosome. $R^2$ (%) corresponds to the percentage phenotypic variation explained by the QTL. The favorable allele is indicated in the last column.

| Trait | Position | Left_Mark | Supp.IV | $R^2$ (%) | fav.allele |
|---|---|---|---|---|---|
| unknown_084 | 5/64 | V.104 | 64-66 | 0.00 | Col-0 |
| unknown_085 | 3/24 | III.54 | 24-26 | 0.00 | Col-0 |
| unknown_086 | 3/50 | III.60 | 50-51 | 0.00 | C24 |
| unknown_086 | 4/22 | IV.75 | 22-26 | 0.00 | Col-0 |
| unknown_086 | 4/42 | IV.75 | 23-51 | 3.74 | Col-0 |
| unknown_089 | 2/48 | II.39 | 43-50 | 3.81 | C24 |
| unknown_091 | 1/65 | I.17 | 61-80 | 2.45 | C24 |
| unknown_091 | 4/6 | IV.70 | 4 - 8 | 22.57 | C24 |
| unknown_091 | 5/9 | V.90 | 6 - 12 | 3.59 | C24 |
| unknown_092 | 3/74 | III.64 | 70 - 76 | 1.74 | Col-0 |
| unknown_092 | 4/8 | IV.73 | 7 - 8 | 42.24 | C24 |
| unknown_092 | 4/32 | IV.75 | 30 - 34 | 0.00 | C24 |
| unknown_092 | 5/8 | V.89 | 4 - 11 | 3.11 | C24 |
| unknown_093 | 4/74 | IV.87 | 72 - 74 | 0.00 | Col-0 |
| unknown_095 | 5/92 | V.111 | 90 - 93 | 0.00 | C24 |
| unknown_096 | 5/76 | V.107 | 74 - 78 | 0.00 | C24 |
| unknown_097 | 3/32 | III.55 | 31 - 33 | 0.00 | C24 |
| unknown_097 | 5/90 | V.111 | 88 - 92 | 0.00 | C24 |
| Urea | 1/76 | I.20 | 74 - 84 | 3.15 | C24 |

Table 3: List of significantly correlated metabolites resulting from pairwise correlations (ordered by p-value). Given are the correlation (COR) and the corresponding p-value (PV). Correlations with a p value below $0.05/182 = 2.76 \cdot 10^{-4}$ (Bonferroni correction) are considered significant.

| METABOLITE | COR | PV |
|---|---|---|
| unknown_038 | 0.23298 | 1.55E-15 |
| unknown_086 | -0.21139 | 5.06E-13 |
| unknown_035 | 0.20551 | 2.25E-12 |
| Citric acid | -0.18815 | 1.41E-10 |
| Ethanolamine | 0.18662 | 2.00-10 |
| Fructose 6-phosphate | -0.18193 | 5.69E-10 |
| Raffinose | -0.17577 | 2.16E-09 |
| Glucose 6-phosphate | -0.16496 | 2.00E-08 |
| Glutamine | -0.16277 | 3.09E-08 |
| Succinic acid | -0.15043 | 3.19E-07 |
| Sinapic acid (cis) | -0.14430 | 9.53E-07 |
| Salicylic acid | -0.13687 | 3.38E-06 |
| unknown_061 | 0.13612 | 3.83E-06 |
| unknown_078 | 0.13446 | 5.03E-06 |
| Tyrosine | -0.13087 | 8.97E-06 |
| Glycerol-3-phosphate | -0.12249 | 3.26E-05 |
| Spermidine (major) | 0.11964 | 4.97E-05 |
| Ornithine | 0.11326 | 1.23E-04 |
| Malic acid | -0.10976 | 2.00E-04 |
| Citramalic acid | -0.10734 | 2.76E-04 |

(continued)

| METABOLITE | COR | PV |
|---|---|---|
| unknown_060 | 0.10558 | 3.47E-04 |
| unknown_071 | -0.10449 | 4.00E-04 |
| unknown_051 | 0.10433 | 4.08E-04 |
| unknown_062 | -0.10156 | 5.81 E-04 |
| Putrescine | 0.10100 | 6.23E-04 |
| unknown_074 | 0.10090 | 6.31 E-04 |
| unknown_005 | -0.10001 | 7.05E-04 |
| Sucrose | -0.09872 | 8.27E-04 |
| unknown_033 | 0.09542 | 1.23E-03 |
| Maleic acid | -0.09279 | 1.68E-03 |
| unknown_043 | 0.08867 | 2.69E-03 |
| unknown_091 | 0.08846 | 2.75E-03 |
| unknown_008 | -0.08711 | 3.19E-03 |
| unknown_088 | -0.08234 | 5.32E-03 |
| Serine (major) | -0.08215 | 5.43E-03 |
| Sinapic acid (trans) | -0.08022 | 6.63E-03 |
| unknown_056 | 0.08020 | 6.64E-03 |
| unknown_048 | -0.07910 | 7.43E-03 |
| unknown_063 | 0.07734 | 8.88E-03 |
| alpha-Tocopherol | -0.07639 | 9.74E-03 |
| Phosphate | -0.07637 | 9.76E-03 |
| Propanoic acid | -0.07606 | 1.01 E-02 |
| unknown_021 | -0.07061 | 1.69E-02 |
| unknown_052 | 0.06812 | 2.12E-02 |
| Ascorbic acid | -0.06728 | 2.29E-02 |
| Benzoic acid | 0.06595 | 2.57E-02 |
| unknown_026 | -0.06558 | 2.66E-02 |
| unknown_049 | 0.06445 | 2.93E-02 |
| unknown_042 | 0.06161 | 3.72E-02 |
| unknown_064 | -0.06090 | 3.94E-02 |
| Glucose-1-phosphate | -0.06065 | 4.03E-02 |
| unknown_080 | -0.05975 | 4.33E-02 |

Table 4: Median correlation between the predicted and true dry weights, the standard deviation of these correlations depending on the size of the training set.

| size of training set | median(COR) | sd(COR) |
|---|---|---|
| 858 | 0.5554561 | 0.04640442 |
| 915 | 0.5535408 | 0.04570179 |
| 972 | 0.5680951 | 0.05756957 |
| 1029 | 0.5733914 | 0.06865319 |
| 1086 | 0.5851052 | 0.08233684 |

Table 5: List of all relevant metabolites determined by the correlation between them and the canonical variate (ordered by absolute correlation). Given are the correlation (COR) and the corresponding p-value (PV).

| METABOLITE | COR | PV |
|---|---|---|
| unknown_038 | 0,3688 | 0,00E+00 |
| unknown_035 | 0,3110 | 0,00E+00 |

(continued)

| METABOLITE | COR | PV |
|---|---|---|
| Ethanolamine | 0,2960 | 0,00E+00 |
| unknown_086 | -0,2738 | 1,51E-24 |
| Fructose 6-phosphate | -0,2449 | 3,66E-16 |
| Citric acid | -0,2373 | 6,12E-18 |
| unknown_078 | 0,2370 | 1,01 E-12 |
| unknown_061 | 0,2241 | 4,52E-13 |
| Glutamine | -0,2227 | 1,68E-12 |
| Glycerol-3-phosphate | -0,2222 | 1,82E-13 |
| Sinapic acid (cis) | -0,2050 | 3,29E-10 |
| Raffinose | -0,1964 | 7,04E-08 |
| Glucose 6-phosphate | -0,1920 | 4,92E-14 |
| Putrescine | 0,1918 | 1,49E-12 |
| Ornithine | 0,1905 | 2,85E-13 |
| unknown_074 | 0,1875 | 9,58E-08 |
| Sucrose | -0,1857 | 7,01E-10 |
| unknown_051 | 0,1851 | 4,81 E-08 |
| unknown_048 | -0,1835 | 8,13E-09 |
| Spermidine (major) | 0,1750 | 7,15E-10 |
| Sinapic acid (trans) | -0,1737 | 7,09E-07 |
| Citramalic acid | -0,1699 | 1,06E-10 |
| Ascorbic acid | -0,1656 | 4,50E-07 |
| Tyrosine | -0,1585 | 1,29E-03 |
| unknown_062 | -0,1544 | 1,67E-08 |
| unknown_071 | -0,1497 | 1,25E-06 |
| Succinic acid | -0,1472 | 5,00E-05 |
| Trehalose | 0,1418 | 3,44E-05 |
| Malic acid | -0,1402 | 2,08E-10 |
| unknown_091 | 0,1377 | 2,20E-06 |
| unknown_060 | 0,1335 | 3,06E-07 |
| unknown_063 | 0,1301 | 6,77E-06 |
| unknown_033 | 0,1284 | 5,46E-06 |
| unknown_054 | -0,1264 | 2,25E-06 |
| Nicotinic acid | 0,1235 | 2,58E-06 |
| unknown_043 | 0,1188 | 3,51 E-05 |
| Propanoic acid | -0,1159 | 5,97E-04 |
| Maleic acid | -0,1154 | 1,71E-06 |
| unknown_079 | 0,1154 | 4,21 E-05 |
| unknown_011 | 0,1140 | 4,34E-03 |
| unknown_021 | -0,1132 | 7,28E-06 |
| Phenylalanine | -0,1105 | 2,16E-04 |
| unknown_084 | 0,1103 | 1,35E-04 |
| unknown_056 | 0,1099 | 9,45E-05 |
| Phosphate | -0,1069 | 7,19E-08 |
| Citrulline | -0,1042 | 1,87E-03 |
| unknown_005 | -0,1030 | 2,99E-05 |
| unknown_088 | -0,1020 | 5,15E-03 |
| unknown_065 | -0,0974 | 1,13E-03 |
| unknown_092 | 0,0965 | 1,86E-03 |
| unknown_019 | -0,0910 | 8,74E-04 |

(continued)

| METABOLITE | COR | PV |
|---|---|---|
| unknown_083 | -0,0910 | 1,05E-03 |
| Urea | -0,0910 | 1,71E-04 |
| unknown_072 | -0,0886 | 1,87E-04 |
| Xylose | 0,0881 | 5,67E-04 |
| Glucose-1-phosphate | -0,0876 | 5,69E-03 |
| unknown_042 | 0,0855 | 3,21 E-03 |
| alpha-Tocopherol | -0,0851 | 2,51 E-03 |
| unknown_013 | -0,0842 | 1,24E-03 |
| unknown_026 | -0,0840 | 2,43E-03 |
| Aspartic acid | -0,0834 | 1,14E-05 |
| unknown_058 | 0,0829 | 1,28E-03 |
| unknown_080 | -0,0815 | 9,88E-04 |
| unknown_030 | -0,0772 | 8,86E-03 |
| Glyceric acid | -0,0765 | 2,02E-04 |
| unknown_070 | -0,0754 | 4,95E-04 |
| Benzene-1,4-dicarboxylic acid | -0,0744 | 5,52E-03 |
| unknown_036 | -0,0721 | 7,98E-03 |
| unknown_064 | -0,0717 | 5,20E-03 |
| unknown_039 | 0,0694 | 3,02E-04 |
| Glutamic acid | -0,0679 | 1,22E-05 |
| unknown_052 | 0,0676 | 6,26E-03 |
| beta-Alanine (3 TMS) | 0,0673 | 2,04E-04 |
| unknown_049 | 0,0663 | 2,19E-03 |
| 4-Aminobutyric acid | 0,0661 | 3,32E-03 |
| unknown_066 | -0,0605 | 9,73E-03 |
| unknown_022 | -0,0582 | 1,29E-03 |
| Threonic acid | -0,0533 | 5,68E-03 |
| unknown_014 | -0,0303 | 4,64E-03 |

**Claims**

1. A method for determining the correlation between the metabolite profiles (MPs) and the expression of a trait of a group of plants comprising the steps of:

   (a) determining the expression of said trait in plants of said group of plants, wherein said plants differ in their expression of said trait;
   (b) determining the MPs of said plants; and
   (c) performing a correlation analysis between said determined expression of said trait and said determined MPs.

2. A method for determining the expression of a trait of a plant comprising the steps of:

   (a) determining the MP of said plant;
   (b) evaluating said MP based on the correlation determined by the method of claim 1; and
   (c) deducing from said evaluation of (b), the expression of said trait exhibited by said plant, wherein said traits are the same.

3. The method of claim 2, wherein said plant belongs to said group of plants.

4. A method for determining the biomass production/growth rate of a plant comprising the steps of:

   (a) determining the MP of said plant;

(b) evaluating said MP based on the results of a correlation analysis between MPs and biomass production/ growth rate of a group of plants; and
(c) deducing from said evaluation of (b) the biomass production/growth rate exhibited by said plant,

5. A method for breeding of a plant comprising the steps of:

   (a) determining the expression of a trait of said plant according to the method of any one of claims 2 to 4; and
   (b) selecting said plant on the basis of the expression of said trait determined by (a).

6. A method for identifying quantitative trait loci (QTLs) for a trait of a group of plants comprising the step of identifying QTLs for metabolite combinations showing strong correlation with the expression of said trait of said group of plants.

7. The method of claim 6, wherein said correlation is determined by the method of claim 1.

8. The method of claim 6, wherein said trait is biomass production/growth rate and wherein said metabolite combinations are deduced from Table 1.

9. A method for identifying a candidate gene involved in the determination of the expression of a trait of a plant comprising the step of
   isolating a gene corresponding to any one of the QTLs as identified by the method of any one of claims 6 to 8.

10. A method for determining the correlation between the MPs and the potential for expression of a trait of a group of plants comprising the steps of:

    (a) determining the potential for expression of said trait of plants of said of said group of plants, wherein said plants differ in their potential for expression of said trait;
    (b) determining the MPs of said plants; and
    (c) performing a correlation analysis between said determined potential for expression of said trait and said determined MPs.

11. A method for predicting the expression of a trait of a plant comprising the steps of:

    (a) determining the MP of said plant;
    (b) evaluating said MP based on the correlation determined by the method of claim 10; and
    (c) deducing from said evaluation of (b), the potential for expression of said trait exhibited by said plant, wherein said traits are the same.

12. The method of claim 11, wherein said plant belongs to said group of plants.

13. A method for predicting the biomass production/growth rate of a plant comprising the steps of:

    (a) determining the MP of said plant;
    (b) evaluating said MP based on the results of a correlation analysis between MPs and the potential for biomass production/growth rate of a group of plants; and
    (c) deducing from said evaluation of (b) the potential for expression of the biomass production/growth rate exhibited by said plant,

14. A method for breeding of a plant comprising the steps of:

    (a) predicting the expression of a trait of said plant according to the method of any one of claims 11 to 13; and
    (b) selecting the plant on the basis of the expression of said trait predicted by (a).

15. A method for identifying QTLs for the potential for expression of a trait of a group of plants comprising the step of identifying QTLs for metabolite combinations showing strong correlation with the potential for expression of said trait of said group of plants.

16. The method of claim 15, wherein said correlation is determined by the method of claim 10.

17. The method of claim 15, wherein said trait is biomass production/growth rate and wherein said metabolite combinations are deduced from Table 1.

18. A method for identifying a candidate gene involved in the determination of the potential for expression of a trait of a plant comprising the step of
isolating a gene corresponding to any one of the QTLs as identified by the method of claim 13.

19. The method of any one of claims 1 to 18, wherein said group of plants is a taxonomic unit.

20. The method of claim 19, wherein said taxonomic unit is species.

21. The method of any one of claims 1 to 18, wherein said group of plants is woody plants.

22. The method of claim 21, wherein said woody plants are trees.

23. The method of claim 22, wherein said trees are slow growing trees.

24. The method of claim 22 or 23, wherein said trees belong to the genus selected from the group consisting of *Quercus; Fagus; Acer* and *Fraxinus.*

25. The method of any one of claims 1 to 24, wherein said correlation analysis comprises a multivariate analysis.

26. The method of any one of claims 1 to 25, wherein said correlation analysis comprises canonical correlation analysis (CCA), ordinary least squares (OLS) regression analysis, partial least squares (PLS) regression analysis, principal component regression (PCR) analysis, ridge regression analysis or least angle regression (LR) analysis, optionally combined with cross validation analysis.

27. The method of any one of claims 1 to 26, wherein said correlation analysis is extended by its non-linear version or supervised machine learning.

28. The method of any one of claims 1 to 27, wherein said correlation analysis comprises the calculation of the highest possible correlation between combinations of metabolite levels extracted from said MPs and said expression or potential for expression of said trait.

29. The method of any one of claims 2 to 9 and 11 to 28, wherein said step of evaluating comprises the fitting of the concentration data of the metabolites of (an) MP(s) into the mathematical model applied for the correlation analysis.

30. The method of claim 29, wherein said result is the highest possible correlation between combinations of metabolite levels extracted from said MPs and said expression of said trait or said potential for expression of said trait.

31. The method of any one of claims 27 to 30, wherein a regression model is used to evaluate said MP.

32. The method of any one of claims 1 to 31, wherein said MP(s) comprise(s) at least 5 metabolites.

33. The method of any one of claims 1 to 32, wherein said MP(s) comprise(s) metabolites of the central carbon or nitrogen metabolism, metabolites of membrane/(phospho)lipid biosynthesis, metabolites of nitrogen assimilation, metabolites of the stress response, metabolites acting as plant hormones, metabolites acting as signals and/or metabolites of the secondary metabolism of plants.

34. The method of any one of claims 1 to 33, wherein said MP(s) comprise(s) metabolites selected from the group consisting of: ethanolamine; fructose-6-phosphate; citric acid; glutamine; glycerol-3-phosphate; sinapic acid (cis); raffinose; ornithine; putrescine; glucose-6-phosphate; spermidine (major); sinapic acid (trans); sucrose; citramalic acid; ascorbic acid; tyrosine; succinic acid; malic acid; trehalose; nicotinic acid; maleic acid; phenylalanine; and salicylic acid.

35. The method of any one of claims 1 to 34, wherein said determination of (an) MP(s) comprises the use of gas chromatography and/or mass spectrometry.

**36.** A method of screening for a plant that exhibits a desired expression or potential for expression of a trait comprising the step of
determining or predicting the expression of said trait according to a method of any one of claims 2 to 4, 11 to 13 and 19 to 35.

**37.** A method of determining whether a treatment influences the expression or potential for expression of a trait of a plant comprising the steps of:

(a) subjecting (a) plant(s) to a treatment;
(b) determining or predicting the expression of said trait according to a method of any one of claims 2 to 4, 11 to 13 and 19 to 35; and
(c) comparing said determined or predicted expression of said trait with the determined or predicted expression of said trait of (a) control plant(s) which has not been subjected to said treatment.

**38.** The method of claim 37, wherein said treatment is selected from the group consisting of light/dark treatments; drought/moisture treatments; heat/cold treatments; substrate composition treatments; treatments with varying space for rooting; treatments with varying macronutrients and/or micronutrients, radiation treatments, treatments with chemicals and treatments with pathogens.

**39.** The method of claim 36, further comprising prior to said step of determining or predicting a step of subjecting plants to a treatment as defined in claim 37 or 38.

**40.** The method of any one of claims 1 to 3, 5 to 12 and 14 to 39, wherein said trait is biomass production/growth rate.

**Figure 1.**

**Figure 2**

Figure 3.

EP 1 936 370 A1

Figure 4.

**Figure 5**

Figure 6

— Biomass
— Biomass_predicted

Critical LOD threshold (alpha = 0.05 or 0.25)

## Figure 7.

European Patent Office

# EUROPEAN SEARCH REPORT

Application Number

EP 06 02 6785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KEURENTJES JOOST J B ET AL: "The genetics of plant metabolism" NATURE GENETICS, vol. 38, no. 7, July 2006 (2006-07), pages 842-849, XP002432091 ISSN: 1061-4036 abstract; pages 846 and 847 | 1-3,6,7, 9-12,15, 16,18-40 | INV. G01N30/72 G01N33/50 |
| Y | | 4,5,13, 14 | |
| X,D | EL-LITHY MOHAMED E ET AL: "Quantitative trait locus analysis of growth-related traits in a new Arabidopsis recombinant" PLANT PHYSIOLOGY (ROCKVILLE), vol. 135, no. 1, May 2004 (2004-05), pages 444-458, XP002432092 ISSN: 0032-0889 abstract; pages 444, 445, 454 | 1-3, 10-14 | |
| Y | | 4,5,13, 14 | TECHNICAL FIELDS SEARCHED (IPC) |
| X | FIEHN O: "Metabolomics: The link between genotypes and phenotypes" PLANT MOLECULAR BIOLOGY, SPRINGER, DORDRECHT, NL, vol. 48, no. 1-2, January 2002 (2002-01), pages 155-171, XP002253436 ISSN: 0167-4412 p. 163, right column; passage bridging paes 167/168 | 1-3 | G01N |

-/--

The present search report has been drawn up for all claims

| Place of search | Date of completion of the search | Examiner |
|---|---|---|
| Munich | 4 May 2007 | Kurz, Birgit |

EPO FORM 1503 03.82 (P04C01)

**EP 1 936 370 A1**

**European Patent Office**

**EUROPEAN SEARCH REPORT**

Application Number

EP 06 02 6785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | ROESSNER UTE ET AL: "Metabolic profiling allows comprehensive phenotyping of genetically or environmentally modified plant systems"<br>PLANT CELL,<br>vol. 13, no. 1, January 2001 (2001-01),<br>pages 11-29, XP002432093<br>ISSN: 1040-4651<br>abstract; pages 12,13, 17, 21, 26<br>----- | 1,10 | |
| A,D | MEYER RHONDA C ET AL: "Heterosis of biomass production in Arabidopsis. Establishment during early development"<br>PLANT PHYSIOLOGY (ROCKVILLE),<br>vol. 134, no. 4, April 2004 (2004-04),<br>pages 1813-1823, XP002432094<br>ISSN: 0032-0889<br>pae 1820, last full paragraph<br>----- | 1-40 | |
| A,D | TOERJEK O ET AL: "Establishment of a high-efficiency SNP-based framework marker set for Arabidopsis."<br>PLANT JOURNAL,<br>vol. 36, no. 1, October 2003 (2003-10),<br>pages 122-140, XP002432095<br>ISSN: 0960-7412<br>abstract; pages 122 and 123<br>----- | 1-40 | **TECHNICAL FIELDS SEARCHED (IPC)** |
| A | KOPKA JOACHIM ET AL: "Metabolite profiling in plant biology: platforms and destinations."<br>GENOME BIOLOGY 2004,<br>vol. 5, no. 6, 2004, page 109, XP002432096<br>ISSN: 1465-6914<br>pages 109.2-3 and 109.7-8<br>----- | 1-40 | |

The present search report has been drawn up for all claims

| Place of search | Date of completion of the search | Examiner |
|---|---|---|
| Munich | 4 May 2007 | Kurz, Birgit |

EPO FORM 1503 03.82 (P04C01)

EP 1 936 370 A1

European Patent
Office

Application Number

EP 06 02 6785

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**European Patent Office**

**LACK OF UNITY OF INVENTION**
**SHEET B**

Application Number

EP 06 02 6785

The Search Division considers that the present European patent application does not comply with the requirements of unity of invention and relates to several inventions or groups of inventions, namely:

1. claims: 1-5, 10-14; 19-35 (partially); 36-40

    Determination of correlation between metabolite profile and a trait
    ---

2. claims: 6-8, 15-17; 19-35 (partially)

            Identification of a QTL for a trait
            ---

3. claims: 9, 18; 19-35 (partially)

            Identification of a gene linked to a QTL
            ---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **TONSOR.** *Plant Cell Environ,* 2005, vol. 28, 2-20
- **CHEN.** *Planta,* 2005, vol. 221, 479-492
- **FERNIE.** *Planta,* 2002, vol. 214, 510-520
- **GIBON.** *Plant Cell,* 2004, vol. 16, 3304-3325
- **ACHARD.** *Science,* 2006, vol. 311, 91-94
- **GIBSON.** *Curr Opin Plant Biol,* 2005, vol. 8, 93-102
- **ROLLAND.** *Biochem Soc,* 2005, vol. T33, 269-271
- **AVONCE.** *Plant Physiol,* 2004, vol. 136, 3649-3659
- **KOLBE.** *P Natl Acad Sci,* 2005, vol. 102, 11118-111123
- **SCHLUEPMANN.** *P Natl Acad Sci,* 2003, vol. 100, 6849-6854
- **THIMM.** *Plant J,* 2004, vol. 37, 914-939
- **STOKES.** *International Sympensium "Heterosis in plants,* May 2006, 18-20
- **SCHAUER.** *Nat Biotechnol,* 2006, vol. 24, 447-454
- **FIEHN.** *Nat Biotechnol,* 2000, vol. 18, 1157-1161
- **LISEC.** Nature Protocols. 2006
- **ROCHA.** *Mamm Genome,* 2004, vol. 15, 83-99
- **SEWALEM.** *Poultry Sci,* 2002, vol. 81, 1775-1781
- **EI-LITHY.** *Plant Physio,* vol. 135, 444-458
- **LI.** *Planta Sci,* 2006, vol. 170, 911-917
- **JANSEN.** *Trends Genet,* 2001, vol. 17, 388-391
- **TÖRJÉK.** *Theoretical & Applied Genetics,* 2006
- **MEYER.** *Plant Physio,* 2004, vol. 134, 1813-1823
- **FIEHN.** *Nature Biotechnology,* 2000, vol. 18, 1157-1161
- **ROESSNER.** *Plant J.,* 2000, vol. 23, 131-142
- **WITTMANN.** *Adv. Biochem. Engin. Biotechnol.,* 2002, vol. 74, 39-64
- **SZYPERSKI.** *Q. Rev. Biophys.,* 1998, vol. 31, 41-106
- **BIRKEMEYER.** *J. Chromotography A,* 2003, vol. 993, 929-937
- **MUELLER.** *Planta,* 2002, vol. 216, 44-56
- **FIEHN.** *Anal.Chem.,* 2000, vol. 72, 3573-3580
- **ROESSNER.** *Plant Cell,* 2001, vol. 13, 11-29

- **JOMPUK.** *J Exp Bot,* 2005, vol. 56, 1153-1163
- **SCHÖN.** *Genetics,* 2004, vol. 167, 485-498
- **HITTALMANI.** *Euphytica,* 2002, vol. 125, 207-214
- **WULLSCHLEGER.** *Can J Forest Res,* 2005, vol. 35, 1779-1789
- **STRASBURGER.** *Lehrbuch der Botanik,* 1991
- *Proceedings of the 5th World Congress on Genetics Applied to Livestock Production: Computing Strategies and Software,* 1994, vol. 22, 65-66
- **BERKS.** *TIBTECH,* 1994, vol. 12, 352-364
- **THIMM.** *Nat Biotechnol,* 2000, vol. 18, 1157-1161
- **TÖRJÉ.** *Plant J,* 2003, vol. 36, 122-40
- **PIEPHO.** *J Agron Crop Sci,* 2003, vol. 189, 310-322
- **LISEC.** *Nature Protocols,* 2006
- **BASTEN.** *Computing Strategies and Software,* 1994, vol. 22, 645-66
- **HACKETT.** *Plant Mol Biol,* 2002, vol. 48, 585-599
- **HOTELLING.** *J Educ Phsychol,* 1935, vol. 26, 139-143
- **KUSS.** *Max Planck Institue for Biological Cybernetics, Technical Report,* 2003, 108
- **WOLD.** Soft modeling by latent variables. Academic Press, 1975
- **HOTELLING,H.** The most predictable criterion. *J Educ Psychol,* 1935, vol. 26, 139-143
- **WOLD.** Soft modelling by latent variables. Academic Press, 1975
- **FRANK.** *Technometrics,* 1993, vol. 35, 109-135
- **FRANK.** *Technometrics,* 1993, vol. 34, 109-135
- **RAZAVI.** *BMC Medical Informatics & Decision Making,* 2005, vol. 5, 29
- **LISO.** *Exp Cell Res,* 1984, vol. 150, 314-320
- **SMIRNOFF.** *Curr Opin Plant Biol,* 2000, vol. 3, 229-235
- **HAGEMAN.** *Mutat Res-Fund Mol M,* 2001, vol. 475, 45-56
- **TKACHENKO.** *Microbiology,* 2001, vol. 70, 422-428
- **GARG.** *P Ntal Acad Sci UUSA,* 2002, vol. 99, 15898-15903

- **JANG.** *Plant Physiol,* 2003, vol. 131, 516-524
- **BASTEN.** *Computing Strategies and Software,* 1994, vol. 22, 65-66
- **EL-LITHY.** *Plant Physiol,* 2004, vol. 135, 444-458
- **LOUDET, O.** *Plant Physiol,* 2003, vol. 131, 345-358
- **RAUH.** *Theor Appl Genet,* 2002, vol. 104, 743-50
- **UNGERER.** *Evolution,* 2003, vol. 57, 2531-2539
- **STEEGE.** *Plant Physiol,* 2005, vol. 139, 1078-1094
- **LI.** *Plant Sci,* 2006, vol. 170, 911-917
- **ATIENZA.** *Euphytica,* 2003, vol. 132, 353-361
- **KEURENTJES.** *Nat Genet,* 2006, 1815
- **KROYMANN.** *Nature,* 2005, vol. 435, 95-98